# EUROPÄISCHE PATENTSCHRIFT

(11) **EP 1 049 801 B1**
(45) Veröffentlichungstag und Bekanntmachung des Hinweises auf die Patenterteilung: **09.08.2006**
(21) Anmeldenummer: 98955519.8
(22) Anmeldetag: 23.10.1998
(51) Int. Cl.: C12Q 1/68

(54) **VERFAHREN ZUR ISOLIERUNG UND REINIGUNG VON NUKLEINSÄUREN AN OBERFLÄCHEN**
METHOD FOR ISOLATING AND PURIFYING NUCLEIC ACIDS ON SURFACES
PROCEDE POUR ISOLER ET PURIFIER DES ACIDES NUCLEIQUES SUR DES SURFACES

(30) Priorität: 23.10.1997 DE 19746874
(43) Veröffentlichungstag der Anmeldung: 08.11.2000
(73) Patentinhaber: Qiagen Gesellschaft mit Beschränkter Haftung, 40724 Hilden (DE)
(72) Erfinder: BASTIAN, Helge, D-40597 Düsseldorf (DE); GAUCH, Simone, D-40597 Düsseldorf (DE); OELMÜLLER, Uwe, D-40699 Erkrath (DE); ULLMANN, Susanne, D-40699 Erkrath (DE)
(74) Vertreter: Zimmermann, Gerd Heinrich
(86) Internationale Anmeldenummer: PCT/EP1998/006756
(87) Internationale Veröffentlichungsnummer: WO 1999/022021

(56) Entgegenhaltungen:
- EP-A- 0 389 063
- EP-A- 0 487 028
- EP-A- 0 649 853
- EP-A- 0 814 156
- WO-A-87/06621
- B. VOGELSTEIN ET AL.: "Preparative and analytical purification of DNA from agarose" PROC. NAT. ACAD. SCI. USA, Bd. 76, Februar 1979, Seiten 615-619, XP000607195 USA in der Anmeldung erwähnt

## Beschreibung

Die vorliegende Erfindung betrifft ein neues Verfahren zur Isolierung und Reinigung von Nukleinsäuren an Oberflächen.

Die Isolierung und Reinigung von Nukleinsäuren aus biologischen und klinischen Probenmaterialien ist von essentieller Bedeutung für Arbeitsbereiche, in denen Nukleinsäure-basierende Arbeitstechniken angewendet werden und in die Nukleinsäure-basierende Techniken gerade Einzug halten. Hierzu zählen zum Beispiel die Vaterschaftsanalyse, Gewebetypisierungen, Identifizierung von Erbkrankheiten, Genomanalyse, molekulare Diagnostik, Bestimmung von Infektionskrankheiten, Tier- und Pflanzenzucht, transgene Forschung, Grundlagenforschung auf dem Gebiet der Biologie und der Medizin sowie zahlreiche verwandte Arbeitsgebiete. Dabei besteht eine generelle Schwierigkeit darin, biologische bzw. klinische Probenmaterialien so aufzubereiten, daß die in ihr enthaltenen Nukleinsäuren direkt in die jeweilige Analysenmethode eingesetzt werden können.

Aus dem Stand der Technik sind bereits zahlreiche Verfahren zur Reinigung von DNA bekannt. So ist es bekannt, Plasmid-DNA beispielsweise zum Zwecke des Klonierens oder auch für andere experimentelle Vorhaben nach dem Verfahren von Birnboim [Methods in Enzymology 100 (1983) 243] zu reinigen. Nach diesem Verfahren wird ein geklärtes Lysat bakteriellen Ursprungs einem Caesiumchlorid Gradienten ausgesetzt und über einen Zeitraum von 4 bis 24 Stunden zentrifugiert. Diesem Verfahrensschritt folgt gewöhnlicherweise die Extraktion und die Praezipitation der DNA. Dieses Verfahren ist mit dem Nachteil verbunden, daß es zum einen apparativ sehr aufwendig und zum anderen sehr zeitaufwendig, kostenintensiv und nicht automatisierbar ist.

Andere Methoden, bei denen geklärte Lysate eingesetzt werden, um DNA zu isolieren, sind die Ionenaustauschchromatographie [Colpan et al., J. Chormatog. 296 (1984) 339] und die Gelfiltration [Moreau et al. Analyt. Biochem. 166 (1987) 188]. Diese Verfahren bieten sich in erster Linie als Ersatz für den Caesiumchlorid-Gradienten an, machen aber ein aufwendiges System für die Lösungsmittelversorgung sowie die Präzipitation der so gewonnenen DNA-Fraktionen erforderlich, da sie gewöhnlicherweise Salze in hoher Konzentration enthalten und sehr verdünnte Lösungen darstellen.

Marko et al. [Analyt. Biochem. 121 (1982) 382] sowie Vogelstein et al. [Proc. Nat. Acad. Sci. 76 (1979) 615] erkannten, daß, falls die DNA aus Nukleinsäure-enthaltenden Extrakten hohen Konzentrationen von Natriumiodid oder Natriumperchlorat ausgesetzt wird, nur die DNA an mechanisch fein zerkleinerten Glass-Scintillationsröhrchen sowie zerkleinerten Glasfasermembranen bzw. Glasfiberplatten bindet, während RNA und Proteine nicht binden. Die so gebundene DNA kann ggf. mit Wasser eluiert werden.

Aus EP 0649835 B1 ist ein Verfahren zur Isolierung von DNA durch Festphasenextraktion an einer modifizierten Glasfasermembran bekannt. Die in diesem Verfahren verwendeten Glasfasermembranen werden z.B. mittels Trifluoroessigsäure und BCl₃ behandelt, um so hydrophile Gruppen, wie z.B. F und elektropositive Atome wie z.B. B oder Si auf der Oberfläche der Glasfasermembran bereitzustellen. Des Weiteren wird in diesem Patent die Lehre offenbart, diese modifizierten Glasfasermembranen in eine Dot-Blot-Apparatur zur Isolierung der Nukleinsäure einzusetzen. Dabei wird eine Membranlage in dieser Apparatur so eingeklemmt, dass sie mehrere Auffanggefäße bedecken kann. Die Nukleinsäure-Lösung wird in die darüber liegenden, passgenauen Reaktionsgefäße eingebracht und die Lösung wird mittels Vakuum durchgesaugt, wobei die Nukleinsäure an der Membran bindet. Nach erfolgter Bindung wird die Membranen mit an bestimmten Stellen der Membran anhaftender Nukleinsäure aus der Apparatur herausgenommen, zurechtgeschnitten, um die einzelnen Nukleinsäurechargen zu trennen und diese dann jeweils in ein Zentrifugengefäß eingebracht. Die Elution erfolgt anschließend mittels eines Elutionspuffers durch Zentrifugation .

In EP 0487028 A2 wird ein weiters Verfahren zur Extraktion und Reinigung von Nukleinsäuren offenbart. Darin wird die die Nukleinsäuren enthaltende Probe lysiert und die Nukleinsäure mittels Alkohol gefällt. Das Verfahren wird in einem Glasfilter durchgeführt und die ausgefällte Nukleinsäure in der Filterschicht immobilisiert. Die in dem Glasfilter festgehaltene Nukleinsäure wird in den nachfolgenden Schritten gewaschen und schließlich die Nukleinsäure eluiert. Dabei wird das Elutionsmittel durch den Glasfilter hindurchgesaugt und dabei die Nukleinsäure aufgelöst. Anschließend wird das gesamte, die Nukleinsäure enthaltende Eluat durch den Glasfilter hindurch in ein Auffanggefäß herausgepresst.

So wird in der WO 87/06621 die Immobilisierung von Nukleinsäuren an einer PVDF-Membran beschrieben. Allerdings werden die an die PVDF-Membran gebundenen Nukleinsäuren anschließend nicht eluiert, sondern die Membran wird samt gebundener Nukleinsäuren direkt in einen PCR-Ansatz eingebracht. Letztendlich wird in dieser internationalen Patentanmeldung und in der weiteren Literatur jedoch die Lehre offenbart, daß hydrophobe Oberflächen bzw. Membranen im allgemeinen zuvor mit Wasser oder Alkohol benetzt werden müssen, um die Nukleinsäuren in halbwegs befriedigenden Ausbeuten immobilisieren zu können.

Für eine Reihe von modernen Applikationen, wie z. B. der PCR-, der Reversed -Transcription-PCR, SunRise, LCX- branched-DNA, NASBA, oder TaqMan-Technologie und ähnlichen Echtzeitquantifizierungsverfahren für PCR, ist es auf der anderen Seite jedoch absolut notwendig, die Nukleinsäuren direkt von der festen Phase lösen zu können. Hierzu ist der WO 87/06621 die Lehre zu entnehmen, daß die Nukleinsäure zwar von den dort eingesetzten Membranen wiedergewonnen werden kann, daß diese Wiedergewinnung jedoch sehr problematisch ist und bei weitem nicht zur quantitativen Isolierung von Nukleinsäuren geeignet ist. Daneben fallen die so gewonnenen Nukeinsäuren in vergleichsweise sehr hoher Verdünnung an - ein Umstand,der weitere Folgeschritte zwecks Konzentrierung und Isolierung zwangsläufig erforderlich macht.

Aus den oben genannten Gründen stellen die aus dem Stand der Technik bekannten Verfahren - insbesondere im Hinblick auf eine Automatisierung des Verfahrensablaufs zur Nukleinsäuregewinnung - keinen geeigneten Ausgangspunkt für eine verfahrenstechnisch möglichst einfache und quantitative Isolierung von Nukleinsäuren dar.

Es ist daher die Aufgabe der vorliegenden Erfindung, die Nachteile der aus dem Stand der Technik bekannten Verfahren zur Isolierung von Nukleinsäuren zu überwinden und ein Verfahren zur Verfügung zu stellen, welches dazu geeignet ist, ohne erheblichen technischen Mehraufwand vollautomatisch durchgeführt werden zu können.

Gelöst wird die Aufgabe erfindungsgemäß durch das Verfahren gemäß dem unabhängigen Patentanspruch 1 und der Verwendung gemäß dem unabhängigegn Patentanspruch 42. Weitere vorteilhafte Aspekte und Ausgestaltungen der Erfindung ergeben sich aus den abhängigen Patentansprüchen, der Beschreibung und den beigefügten Zeichnungen.

Dabei ist die Erfindung auf ein Verfahren gerichtet, das Membranen, z. B. poröse Membranen verwendet, an welche die Nukleinsäuren auf einfache Weise aus der die Nukleinsäuren enthaltenden Probe immobilisiert und mittels ebenso einfacher Verfahrensschritte wieder abgelöst werden können, wobei es die erfindungsgemäß einfache Prozeßführung ermöglicht, das Verfahren insbesondere vollautomatisch durchführen zu können.

Ein weiterer Aspekt der vorliegenden Erfindung ist darauf gerichet, Nukleinsäuren an eine Membran, in der Art und Weise zu binden, daß sie in einem folgenden Reaktionsschritt ohne weiteres wieder von dieser Phase abgelöst werden können und ggf. in weiteren Anwendungen - wie z. B. Restriktionsverdauung, RT, PCR oder RT-PCR oder in jedweder anderen oben genannten geeigneten Analyse- bzw. Enzymreaktion eingesetzt werden können.

Die Erfindung stellt ein Verfahren zur Isolierung von Nukleinsäuren mit den folgenden Schritten bereit:
- Beschicken einer Membran aus einer Richtung mit Nukleinsäuren;
- Immobilisieren der Nukleinsäuren an der Membran;
- Ablösen der immobilisierten Nukleinsäuren von der Membran; und
- Abnehmen der abgelösten Nukleinsäuren von derselben Seite der Membran von der sie der Membran zugeführt wurde, wobei die Isolierung der Nukleinsäure an der in dem Gefäß fixierten Membran erfolgt.

Vorzugsweise erfolgt das Beschicken von oben. In diesem Fall kann die Gravitation zum Sammeln eines Puffers, der zum Ablösen verwendet wird, und zum Ablösen verwendet werden. Zwischen dem Immobilisierungs- und dem Ablöseschritt kann ein Waschen der immobilisierten Nukleinsäuren mit zumindest einem Waschpuffer erfolgen. Das Waschen umfasst für jeden Waschpuffer vorzugsweise folgende Schritte:
- Aufbringen einer vorbestimmten Menge an Waschpuffer auf die Membran, und
- Durchsaugen des Waschpuffers durch die Membran.

Das Beschicken und Immobilisieren der Nukleinsäuren kann wiederum folgende Schritte umfassen:
- Mischen der Nukleinsäuren mit einem Immobilisierungspuffer,
- Beschicken der Nukleinsäuren mit dem Immobiliserungspuffer auf die Membran, und
- Durchsaugen der füssigen Bestandteile durch die Membran.

Das Verfahren weist den großen Vorteil auf, leicht automatisierbar zu sein, so daß zumindest einer der Schritte durch einen Automaten vollautomatisch durchgeführt werden kann. Ebenso ist es möglich, daß alle Schritte des Verfahrens durch einen Automaten in gesteuerter Abfolge durchgeführt werden.

Speziell in diesen Fällen, aber auch bei manueller Bearbeitung ist es möglich, daß eine Mehrzahl von Nukleinsäuren gleichzeitig der Isolierung unterworfen werden.

Schließlich können in dem erfindungsgemäßen Verfahren zwischen dem Ablöse- und dem Abnehmschritt zumindest einmal folgende Schritte durchgeführt werden:
- Durchführen zumindest einer chemischen Reaktion an den Nukleinsäuren;
- Immobilisieren der Nukleinsäuren an der Membran; und
- Ablösen der immobilisierten Nukleinsäuren von der Membran mit einem Elutionsmittel.

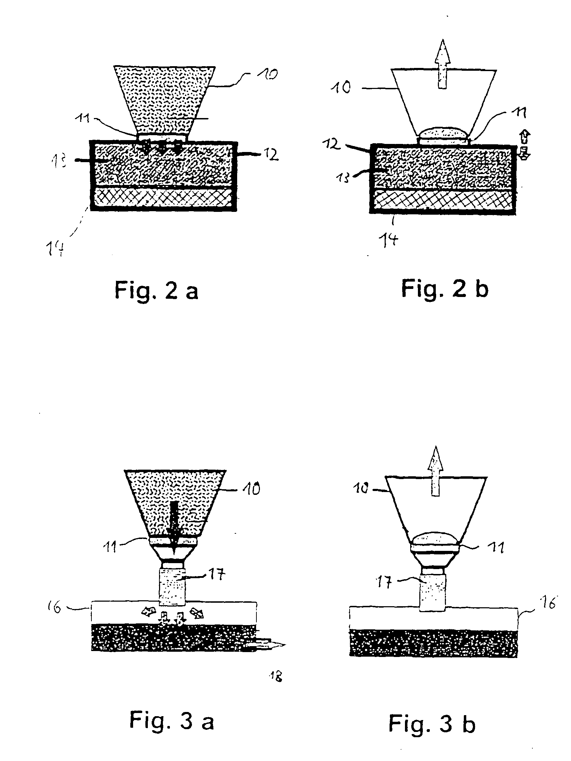

Wie vorstehend skizziert, wird die Nukleinsäure von der Membran im wesentlichen in derselben Richtung eluiert (abgelöst), in der sie aufgetragen und immobilisiert worden ist. Unter "derselben Richtung" ist dabei im Grunde genommen jede Richtung unter einem Winkel von kleiner oder gleich 180° zu verstehen, so daß bei der Eluierung die Nukleinsäuren jedenfalls nicht die Membran, durchdringen, sondern in Gegenrichtung der Beschickungsrichtung von der Membran entfernt werden, in der sie auf die Membran aufgebracht worden sind. In bevorzugten Ausführungsformen werden demgegenüber die anderen Puffer, also derjenige Puffer, in dem sich die Nukleinsäuren beim Beschicken befinden, und ggfs. ein Waschpuffer, durch die Membran durchgesaugt oder sonstwie transferiert. Wenn die Isolierung an einer in einem Gefäß befindlichen Membran erfolgt, wobei die Membran den gesamten Querschnitt des Gefässes ausfüllt, ist die bevorzugte Beschickung von oben. Der Abnahmeschritt erfolgt in diesem Fall wiederum nach oben. Fig. 2 zeigt beispielsweise ein trichterförmiges Isoliergefäss, das von oben beschickt wird und bei dem die Abnahme der Nukleinsäuren nach oben erfolgt.

Es versteht sich jedoch, daß auch andere Anordnungen denkbar sind, so z. B. eine Abnahme der Nukleinsäuren von unten. Es ist beispielsweise vorstellbar, daß ein Nukleinsäuren enthaltender Puffer wie ein Lysatpuffer mittels einer Saugvorrichtung aus einem Reaktionsgefäß unmittelbar in ein Isoliergefaß gesaugt wird, so daß sich die Nukleinsäuren an die Unterseite einer Membran in dem Isoliergefäß binden. In einem solchen Fall könnte die Abnahme der Nukleinsäuren von der Membran dadurch erfolgen, daß ein Eluierpuffer von unten aufgesaugt wird und nach Ablösen der Nukleinsäuren wiederum nach unten in ein Gefäss abgelassen wird. Hierbei erfolgt also die Abnahme der Nukleinsäuren nach unten.

Auch eine seitliche Abnahme der Nukleinsäuren ist möglich, beispielsweise wenn eine flachliegende Säule mit einer darin angeordneten Membran im Durchflußverfahren mit einem Lysat beshickt wird und im Anschluß die liegende Säule auf der Seite der Membran, an der die Nukleinsäuren binden, mit Eluierpuffer gespült wird.

Ein Beispiel für den maximal möglichen Winkel von 180° ist eine Schräge mit einer zur Bindung von Nukleinsäuren geeigneten Membran über welche die verschiedenen Lösungen bzw. Puffer herabfließen. Wie alle Puffer, kommt auch der Eluierpuffer von einer Seite und fließt zur anderen Seite ab. In diesem Fall bilden Einströmrichtung des Puffers und Abströmrichtung des Puffers mit den darin aufgenommenen Nukleinsäuren einen Winkel von 180°, die Abnahme erfolgt jedoch immer noch auf derselben Seite der Membran wie die Immobilisierung.

Unter Nukleinsäure im Sinne der vorliegenden Erfindung sollen dabei alle wäßrigen oder sonstigen Lösungen von Nukleinsäuren und ebenso alle Nukleinsäuren enthaltenden biologischen Materialien und biologischen Proben verstanden werden. Dabei wird im Sinne der vorliegenden Erfindung eine Nukleinsäure enthaltende Probe oder ein Material durch eine Probe bzw. einen Probenansatz definiert, die bzw. der Nukleinsäuren enthält, die als geeignete Edukte für in vitro Transkriptionen, PCR-Reaktionen, oder cDNA-Synthesen dienen können - Unter biologischem Material bzw. biologischer Probe sollen dabei z. B. Plasma, Körperflüssigkeiten - wie beispielsweise Blut, Sputum, Urin, Faeces, Sperma, - Zellen, Serum, Leukozytenfraktionen, Crusta Phlogistica, Abstriche, Gewebeproben jeder Art, Pflanzen und Pflanzenteile, Bakterien, Viren, Hefen etc., wie sie beispielsweise in der Europäischen Patentanmeldung Nr. 95909684.3 offenbart sind, auf die hiermit inhaltlich Bezug genommen wird - oder auch freie Nukleinsäuren fallen. Unter Nukleinsäuren fallen im Sinne der vorliegenden Erfindung alle möglichen Arten von Nukleinsäuren, wie z. B. Ribonukleinsäuren (RNA) und Desoxyribonukleinsäuren (DNA) in allen Längen und Konfigurationen, wie Doppelstrang, Einzelstrang, circulär und linear, verzweigt etc.; monomere Nukleotide, Oligomere, Plasmide, virale und bakterielle DNA und RNA, sowie genomische oder sonstige nichtgenomische DNA und RNA aus Tier- und Pflanzenzellen oder anderen Eukaryoten, t-RNA, mRNA in prozessierter und unprozessierter Form, hn-RNA, rRNA und cDNA sowie alle anderen, denkbaren Nukleinsäuren.

Nach dem erfindungsgemäßen Verfahren nimmt man die oben beschriebene, Nukleinsäuren enthaltende Probe in einer Lösung auf, die geeignete Salze und/oder Alkohol(e) enthält, anschließend ggf. den Ansatz aufschließt und mischt und die so erhaltene Mischung mittels eines Vakuums, auf dem Wege einer Zentrifugation, mittels Überdruck, durch Kapillarkräfte oder durch andere geeignete Verfahren durch eine poröse Membran führt, wobei die Nukleinsäuren an der Membran immobilisiert werden.

Als Salze für das Immobilisieren von Nukleinsäuren an Membranen kommen Salze von Alkali- oder Erdalkalimetallen mit Mineralsäuren in Frage; insbesondere Alkali- oder Erdalkalihalogenide bzw. -sulfate worunter die Halogenide des Natriums oder Kaliums oder Magnesiumsulfat besonders bevorzugt werden.

Ferner sind zur Durchführung des erfindungsgemäßen Verfahrens Salze von ein- oder mehrbasischen oder auch polyfunktionellen organischen Säuren mit Alkali- oder Erdalkalimetallen geeignet. Darunter fallen insbesondere Salze des Natriums, des Kaliums oder des Magnesiums mit organischen Dicarbonsäuren - wie z. B. Oxal-, Malon- oder Bernsteinsäure - oder mit Hydroxy- bzw. Polyhydroxycarbonsäuren - wie z. B. bevorzugterweise mit Zitronensäure.

Als besonders zweckmäßig hat sich dabei der Einsatz von sog. chaotropen Agenzien herausgestellt. Chaotrope Substanzen sind in der Lage, die dreidimensionale Struktur der Wasserstoffbrückenbindung zu stören. Hierdurch werden auch die intramolekularen Bindungskräfte geschwächt, die bei der Ausbildung der räumlichen Strukturen, wie z. B. Primär-, Sekundär-, Tertiär- oder Quartärstrukturen, bei biologischen Molekülen beteiligt sind. Geeignete chaotrope Agenzien sind dem Fachmann aus dem Stand der Technik hinlänglich bekannt [Römpp, Lexikon der Biotechnologie, Herausgeber. H. Dellweg, R.D. Schmid u. W.E. Fromm, Thieme Verlag, Stuttgart 1992].

Als bevorzugte chaotrope Substanzen gelten gemäß der vorliegenden Erfindung beispielsweise Salze aus der Gruppe der Trichloracetate, Thiocyanate, Perchlorate, Jodide oder Guanidinium-Hydrochlorid und Harnstoff.

Die chaotropen Substanzen werden dabei in 0,01 bis 10 molarer wässeriger Lösung, bevorzugt in 0,1 bis 7 molarer wässeriger Lösung und besonders bevorzugt in 0,2 bis 5 molarer wässeriger Lösung eingesetzt. Hierbei können die vorbezeichneten chaotropen Agenzien allein oder in Kombinationen verwandt werden. Insbesondere werden 0,01 bis 10 molare wässerige Lösungen, bevorzugt 0,1 bis 7 molare wässerige Lösungen und besonders bevorzugt in 0,2 bis 5 molare wässerige Lösungen von Natriumperchlorat, Guanidinium-Hydrochlorid, Guanidinium-isothiocyanat, Natriumiodid und/oder Kaliumiodid eingesetzt.

Als Alkohole kommen für die Durchführung des erfindungsgemäßen Verfahrens zunächst alle Hydroxylderivate von aliphatischen oder acyclischen gesättigten oder ungesättigten Kohlenwasserstoffen in Betracht. Dabei ist es zunächst unerheblich, ob diese Verbindung eine, zwei, drei oder mehr Hydroxylgruppen - wie mehrwertige C₁-C₅-Alkanole, beispielsweise Ethylenglykol, Propylenglykol oder Glycerin - enthalten.

Daneben zählen ebenfalls die Zuckerabkömmlinge, die sog. Aldite, wie auch die Phenole - beispielsweise Polyphenole - zu den erfindungsgemäß einsetzbaren Alkoholen.

Unter den vorgenannten Hydroxyverbindungen werden die C₁-C₅₋Alkanole - wie Methanol, Ethanol, n-Propanol, *tert*.-Butanol und die Pentanole besonders bevorzugt.

Die Immobilisierung kann unter sauren, neutralen oder basischen Bedingungen durchgeführt werden. So kann der pH bei der Immobilisierung zwischen 3 und 11 liegen, vorzugsweise wird bei einem pH von 4 bis 8 immobilisiert. Wenn RNA isoliert werden soll, liegt der pH vorzugsweise eher im neutralen Bereich, während bei DNA-Isolierungen ein saurer pH günstiger sein kann. So kann der pH für RNA-Isolierungen beispielsweise bei 6 bis 8 liegen, bevorzugterweise bei 6,5 bis 7,5. Für DNA-Isolierungen liegt der pH günstigerweise bei 4 bis 8, vorzugsweise bei 4 bis 6.

Als hydrophil gelten im Sinne der vorliegenden Erfindung solche Stoffe bzw. Membranen, die von ihrem chemischen Charakter her sich leicht mit Wasser mischen bzw. Wasser aufnehmen.

Als hydrophob gelten im Sinne der vorliegenden Erfindung solche Stoffe bzw. Membranen, die von ihrem chemischen Charakter her nicht in Wasser eindringen - bzw. vice versa - und die nicht darin zu bleiben vermögen.

Unter einer Oberfläche wird im Sinne der vorliegenden Erfindung jede mikroporöse Trennschicht verstanden. Im Falle einer Membran wird die Oberfläche durch eine Folie aus einem polymeren Material gebildet. Das Polymer wird bevorzugt aus Monomeren mit polaren Gruppen aufgebaut.

Bei Verwendung hydrophober Membranen gelten als bevorzugt im Sinne der vorliegenden Erfindung Membranen, die aus einem hydrophilen Grundmaterial bestehen und die durch eine entsprechende chemische Nachbehandlung, die an sich aus dem Stand der Technik bekannt ist - hydrophobisiert wurden, wie z. B. hydrophobisierte Nylon-Membranen, die kommerziell erhältlich sind.

Unter hydrophobisierten Membranen werden erfindungsgemäß allgemein solche Membranen verstanden, die als unter Umständen ursprünglich hydrophile Membran mit den unten erwähnten Hydrophobisierungsmitteln überzogen wurden. Derartige Hydrophobisierungsmittel überziehen hydrophile Substanzen mit einer dünnen Schicht hydrophober Gruppen, wozu beispielsweise längere Alkylketten oder Siloxangruppen gehören. Geeignete Hydrophobisierungsmittel sind aus dem Stand der Technik in großer Zahl bekannt und stellen erfindungsgemäß Paraffine, Wachse, Metallseifen etc. ggf. mit Zusätzen an Aluminium bzw. Zirkoniumsalzen, quartäre organische Verbindungen, Harnstoffderivate, fettstoffmodifizierte Melaminharze, Silicone, zinkorganische Verbindungen, Glutardialdehyde und ähnliche Verbindungen dar.

Daneben gelten als erfindungsgemäß einsetzbare hydrophobe Membranen solche Membranen, die hydrophobisiert sind und deren Grundmaterial polare Gruppen aufweisen kann. Gemäß dieser Kriterien eignen sich beispielsweise - insbesondere hydrophobisierte - Materialien aus der folgenden Gruppe für den erfindungsgemäßen Einsatz:

Nylon, Polysulfone, Polyethersulfone, Polycarbonate, Polyacrylate sowie Acrylsäurecopolymere, Polyurethane, Polyamide, Polyvinylchlorid, Polyfluorocarbonate, Polytetrafluoroethylen, Polyvinylidenfluorid, Polyvinylidendifluorid, Polyethylentetrafluoroethylen-Copolymerisate, Polyethylenchlorotrifluoroethylen-Coplymerisate oder Polyphenylensulfid sowie Cellulose und Cellulose-Mischester oder Nitrocellulosen wie auch hydrophobisierte Glasfasermembranen, worunter hydrophobisierte Nylon-Membrane besonders bevorzugt sind.

Bevorzugte hydrophile Membranen umfassen per se hydropile Materialien und auch hydrophobe Materialien, die hydrophilisiert worden sind. Beispielsweise können verwendet werden hydrophiles Nylon, hydrophile Polyethersulfone, hydrophile Polycarbonate, Polyester, hydrophile Polytetrafluoroethylene auf Polypropylengeweben, hydrophile Polytetrafluoroethylene auf Polypropylenvliesen, hydrophilisierte Polyvinylidenfluoride, Polyvinylidendifluoride, hydrophilisierte Polytetrafluorethylene und hydrophile Polyamide.

Die Membranen, die im erfindungsgemäßen Verfahren eingesetzt werden, haben dabei beispielsweise einen Porendurchmesser von 0,001 bis 50 µm, vorzugsweise 0,01 bis 20 µm und besonders bevorzugt 0,05 bis 10 µm.

Als Waschpuffer kommen ebenfalls die oben beschriebenen Salze oder Alkohole bzw. Phenole oder Polyphenole in Frage. Die Temperaturen liegen im Waschschritt in einem Intervall von üblicherweise 10° bis 30 °C, wobei auch höhere Temperaturen erfolgreich angewandt werden können.

Zur Elution der gebundenen Nukleinsäure eignen sich erfindungsgemäß Wasser oder wässerige Salzlösungen als Elutionsmittel. Als Salzlösungen werden Pufferlösungen eingesetzt, die aus dem Stand der Technik bekannt sind, wie beispielsweise Morpholinopropansulfonsäure (MOPS), Tris(hydroxymethyl)aminomethan (TRIS), 2-[4-(2-Hydroxyethyl)-1-piperazino]ethansulfonsäure (HEPES) in einer Konzentration von 0,001 bis 0,5 Mol/Liter, bevorzugt 0,01 bis 0,2 Mol/Liter, besonders bevorzugt 0,01 bis 0,05 molare Lösungen. Daneben werden bevorzugt wässerige Lösungen von Alkali- oder Erdalkalimetallsalzen, insbesondere deren Halogenide, eingesetzt, darunter 0,001 bis 0,5 molare, bevorzugt 0,01 bis 0,2 molare, besonders bevorzugt 0,01 bis 0,05 molare wässerige Lösungen von Natriumchlorid, Lithiumchlorid, Kaliumchlorid oder Magnesiumdichlorid. Daneben können bevorzugt auch Lösungen von Salzen der Alkalimetalle oder Erdalkalimetalle mit Carbon- oder Dicarbonsäuren wie Oxalsäure oder Essigsäure, wie Lösungen von Natriumacetat oder -oxalat in Wasser eingesetzt werden, beispielsweise in dem zuvor genannten Konzentrationsbereich, wie z. B. 0,001 bis 0,5 molare, bevorzugt 0,01 bis 0,2 molare, besonders bevorzugt 0,01 bis 0,05 molare Lösungen.

Ganz besonders wird reines Wasser als Elutionsmittel bevorzugt, beispielsweise demineralisiertes, bidestiliertes, oder Millipore-Wasser.

Die Eluierung kann bei Temperaturen von 10° bis 70°C, bespielsweise bei 10° bis 30°C oder auch bei höheren Temperaturen erfolgreich durchgeführt werden. Auch ein Eluieren mit Wasserdampf ist möglich.

Hinsichtlich der einzelnen Schritte wird das erfindungsgemäße Verfahren wie folgt durchgeführt:

Das Lysat der zur Gewinnung der Nukleinsäuren dienenden Probe oder die ursprünglich freie(n) Nukleinsäure(n) wird/werden beispielsweise in eine (Plastik-)Säule pipettiert, in der - beispielsweise auf dem Boden - die Membran fixiert wird. Zweckmäßigerweise kann die Membran auf einer Fritte fixiert werden, die als mechanische Unterstützung dient. Anschließend wird das Lysat durch die Membran geführt, was durch Anlegen eines Vakuums am Ausgang der Säule erreicht werden kann. Auf der anderen Seite kann der Transport durch einen Lysat-seitigen Überdruck erfolgen. Daneben kann - wie schon zuvor erwähnt - der Lysattransport auf dem Wege der Zentrifugation oder durch die Einwirkung von Kapillarkräften bewerkstelligt werden; letzteres kann zum Beispiel mit einem schwammartigen Material geschehen, das unterhalb der Membran mit dem Lysat bzw. Filtrat in Kontakt gebracht wird.

Der in bevorzugten Ausführungsformen eingefügte Waschschritt, kann erfolgen, indem der Waschpuffer durch die Membran hindurchtransportiert wird oder auf derselben Seite der Membran verbleibt wie die Nukleinsäuren. Wird der Waschpuffer hindurchtansportiert bzw. gesaugt, so kann dies auf unterschiedliche Weisen geschehen, z. B. durch einen auf der anderen Seite der Membran angeordneten Schwamm, eine Saug- oder Überdruckvorrichtung oder durch Zentrifugation oder Gravitation.

Der Vorteil einer Anordnung mit einem schwammartigen Material besteht in einer einfachen, sicheren und bequemen Möglichkeit der Filtrat-Entsorgung - es muß in diesem Fall nur der Schwamm, der nunmehr mit dem Filtrat mehr oder weniger vollgesogen ist, ausgetauscht werden. Es wird an dieser Stelle deutlich, daß die Säule kontinuierlich oder auch batch-weise betrieben werden kann und, daß beide Betriebsarten vollautomatisiert durchgeführt werden können, bis die Membran mit Nukleinsäure gesättigt ist. In letzten Schritt erfolgt die Elution der Nukleinsäure, welche beispielsweise von der Membran abpipettiert bzw. abgehoben oder in sonstiger Weise nach oben entfernt werden kann. Maßgeblich für den Eluierschritt im erfindungsgemäßen Verfahren ist jedenfalls, daß die Nukleinsäuren von derselben Seite der Membran abgenommen werden, von der sie der Membran auch zugeführt wurden, daß also kein Durchtritt der Nukleinsäuren durch die Membran zu erfolgen hat. Eine solche Verfahrensanordnung ermöglicht es, alle nicht mehr benötigten Flüssigkeiten, wie den urspünglichen Lysepuffer und die Waschpuffer, auf eine "Abfallseite" zu saugen oder durch Gravitation dorthin zu bringen, während das Eluat auf der anderen Seite bleibt. Eine solche Anordnung ermöglicht in besonders einfacher Weise eine Automatisierung des erfindungsgemäßen Verfahrens, da für Zugabe des Lysates und Abnahme des Eluats nur auf einer Seite der Oberflächen eine Pipettiervorrichtung vorgesehen sein muß, die andere Seite der Oberfläche hingegen keinen "Reinbereich" enthalten muß. Somit lässt sich auch durch die räumliche Trennung eine kontaminationsfreie Isolierung von Nukleinsäuren, insbesondere RNase-Freiheit, in einfachster Weise sicherstellen. Zudem müssen die Isoliergefäße, z. B. Reinigungssäulen, nicht repositioniert werden, um einerseits Abfall verwerfen zu können, andererseits das Eluat durch dieselbe Öffnung des Gefässes aufzufangen. Auch dies bedeutet eine Vereinfachung der Automatisierung.

Das Auffangen von Fraktionen, welche in großer Verdünnung die gewünschten Nukleinsäuren enthalten, und das anschließende Konzentrierung erfordert, entfällt bei dem erfindungsgemäßen Verfahren völlig. Vielmehr fallen die gewünschten Nukleinsäuren in schwach oder nicht-salzhaltigen Lösungen in sehr kleinen Volumina an, was von großem Vorteil für alle molekularbiologischen Analyseverfahren ist, da hier gewünscht ist, reine Nukleinsäuren in möglichst kleinen Volumina bei gleichzeitig hoher Konzentration einzusetzen. Um möglichst kleine Volumina an Eluat erzielen zu können, werden als Oberflächen insbesondere Membranen bevorzugt, die möglichst dünn sind, so daß sich nur wenig Flüssigkeit in ihnen ansammeln kann. Weniger bevorzugt sind hingegen Vliese wie z. B. Silicagelvliese, da diese ein relativ großes Volumen an Eluat aufzunehmen vermögen, was das Abnehmen des Eluats nach oben erschwert und das notwendige Eluatvolumen in ungünstiger Weise steigert.

Ferner bietet die vorliegende Erfindung den Vorteil, daß bei einer vertikalen Anordnung des Gefässes (Membran dann horizontal orientiert) das über der Membran befindliche Volumen als Reaktionsraum genutzt werden kann. So ist es z. B. möglich, nach dem Isolieren und Ablösen der nach dem erfindungsgemäßen Verfahren gewonnenen Nukleinsäuren, diese zunächst nicht abzunehmen, sondern im Isoliergefäß zu belassen und einer molekularbiologischen Applikation - wie Restriktionsverdauung, RT, PCR, RT-PCR oder Enzymreaktionen - zu unterwerfen, die aus diesen Reaktionen hervorgehenden Nukleinsäuren gemäß dem erfindungsgemäßen Verfahren erneut an die Membran zu binden, ggfs. wie beschrieben zu waschen und anschließend zu eluieren, zu isolieren, bzw. zu analysieren, beispielsweise mittels Spektroskopie, Fluorometrie oder ähnlichen Meßverfahren.

Die erfindungsgemäß isolierten Nukleinsäuren sind frei von nukleinsäureabbauenden Enzymen und haben eine derartig hohe Reinheit, daß sie unmittelbar in verschiedensten Weisen weiterbehandelt und bearbeitet werden können.

Die erfindungsgemäß hergestellten Nukleinsäuren können für Klonierungen verwendet werden und als Substrate für verschiedenste Enzyme dienen, wie beispielsweise DNA-Polymerasen, DNA-Restriktionsenzyme, DNA-Ligase und reverse Transkriptase.

Die durch das erfindungsgemäße Verfahren bereitgestellten Nukleinsäuren eignen sich in besonders guter Weise zur Amplifikation, insbesondere für die PCR, Strand Displacement Amplifikation, Rolling Circle Verfahren, Ligase Chain Reaction (LCR) und ähnlicher Verfahren.

Das erfindungsgemäße Verfahren eignet sich weiterhin in besonders guter Weise zur Bereitstellung von Nukleinsäuren für die Verwendung in der Diagnostik, insbesondere für ein Diagnoseverfahren, welches dadurch gekennzeichnet ist, daß die durch das erfindungsgemäße Verfahren gereinigte Nukleinsäure in einem Folgeschritt amplifiziert wird und anschließend und/oder gleichzeitig die so amplifizierte Nukleinsäure detektiert wird (z. B. Holland, P.M. et al., 1991. Proc. Natl. Acad. Sci. 88, 7276 - 7280. Livak, K.J. et al., 1995. PCR Methods Applic. 4, 357 - 362; Kievits, T. et al., 1991. J. Virol. Meth. 35, 273 - 286; Uyttendaele, M. et al., 1994. J. Appl. Bacteriol. 77, 694 - 701).

Desweiteren eignet sich das erfindungsgemäße Verfahren in besonders guter Weise zur Bereitstellung von Nukleinsäuren, welche in einem Folgeschritt einem auf einer Hybridisierungsreaktion basierenden Signalamplifikationsschritt unterzogen werden, insbesondere dadurch gekennzeichnet, daß die durch das erfindungsgemäße Verfahren bereitgestellten Nukleinsäuren mit "Verzweigten Nukleinsäuren", insbesondere Branched DNA und/oder Branched RNA und/oder entsprechende Dendrimer Nukleinsäuren, wie in den folgenden Literaturstellen beschrieben (z. B. Bresters, D. et al., 1994. J. Med. Virol. 43 (3), 262 - 286; Collins M.L. et al., 1997. Nucl. Acids Res. 25 (15), 2979 - 2984;), in Kontakt gebracht werden und das entstehende Signal detektiert wird.

Im folgenden wird ein Beispiel für die Automatisierbarkeit des erfindungsgemäßen Verfahrens erläutert und werden Beispiele für die Durchführung des Verfahrens mit verschiedenen Oberflächen und Nukleinsäuren angegeben. Hierbei wird Bezug genommen auf die beigefügten Zeichnungen, in denen folgendes dargestellt ist.
Fig. 1 zeigt einen zur Durchführung des erfindungsgemäßen Verfahrens geeigneten Automaten in einer schematisierten Darstellung.
Fig. 2 zeigt eine erste Ausführungsform eines Isoliergefässes und Abfallbehälters zur Durchführung des erfindungsgemäßen Verfahrens.
Fig. 3 zeigt eine zweite Ausführungsform eines Isoliergefässes und Abfallbehälters zur Durchführung des erfindungsgemäßen Verfahrens.
Fig. 4 zeigt eine dritte Ausführungsform eines Isoliergefässes und Abfallbehälters zur Durchführung des erfindungsgemäßen Verfahrens.
Fig. 5 zeigt die Absorption einer Probe im Bereich von 220 bis 320 nm.
Fig. 6 zeigt das Ethidiumbromid-gefärbte Gel einer elektrophoretischen Auftrennung verschiedener Proben nach dem erfindungsgemäßen Verfahren.
Fig. 7 zeigt ein weiteres Ethidiumbromid-gefärbtes Gel einer elektrophoretischen Auftrennung verschiedener Proben nach dem erfindungsgemäßen Verfahren.

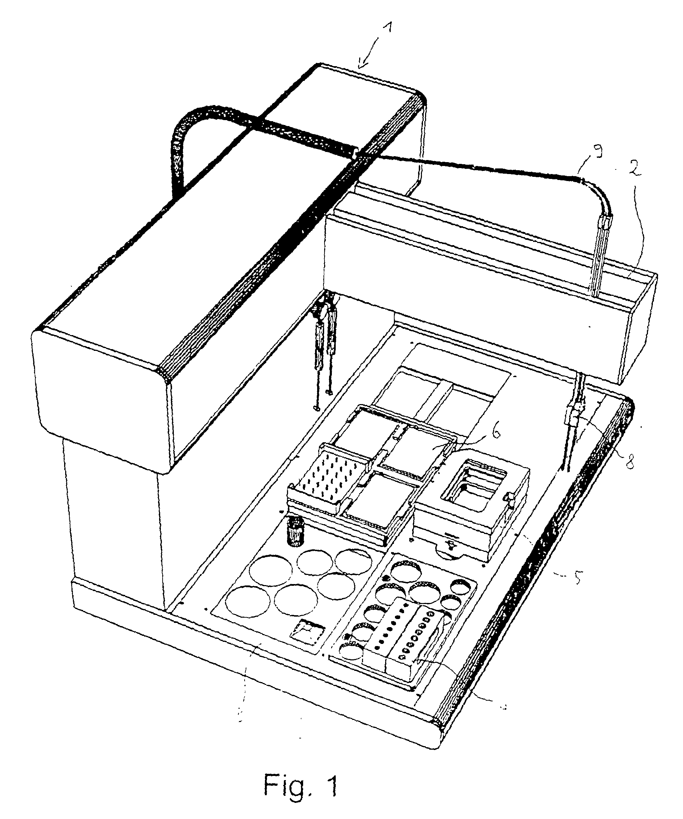

Das erfindungsgemäße Verfahren wird vorzugsweise teilweise oder vollständig, d.h. in allen seinen Schritten, automatisiert durchgeführt. Ein Beispiel für einen geeigneten Automaten ist in Fig. 1 dargestellt, bei dem ein Hauptteil 1 mit Steuerungselektronik und Antriebsmotoren mit einer Arbeitsplattform 3 und einem fahrbaren Arm 2 ausgestattet ist. Auf der Arbeitsplattform sind verschiedene Elemente positioniert, so Bereiche 4 zur Halterung von verschiedenen Gefässen. Eine Absaugvorrichtung 5 dient zum Absaugen von Flüssigkeiten aus darüber positionierten, nach unten offenen Isoliergefässen, oder sonst mit der Absaugvorrichtung verbundenen Gefässen. Ein Rüttler 6 ist ebenfalls vorgesehen, der beispielsewise zum Lysieren von biologischen Proben verwendet werden kann. Die verwendeten Anordnungen von Isoliergefässen sind beispielsweise Spritzgußteile mit integrierten Isoliergefässen, in die erfindungsgemäße Oberflächen eingelegt sind. Es können typischerweise 8, 12, 24, 48, 96 oder bis zu 1536 Isoliergefässe vorgesehen sein, wie sie z.B. in den Formaten moderner Multi-Well-Platten zur Verfügung gestellt werden. Auch noch höhrere Isoliergefäßzahlen in einer Anordnung sind vorstellbar, wenn entsprechende Standards zur Verfügung stehen. Mit Hilfe von Luer-Adaptern ist es jedoch auch möglich, die Böden der Anordnungen separat bereitzustellen und je nach Bedarf mit einem oder mehreren Isoliergefässen zu bestücken. Auch einzeln gearbeitete Isoliergefässe ohne Luer-Adapter werden von der Erfindung miterfasst.

Unter eine Saug- und Dispensiervorrichtung 8 werden die Anordnungen von Isoliergefässen in den Automaten eingesetzt und über diese können Flüssigkeiten aufgenommen und abgegeben werden. Hierbei können mehrere einzelne Saugrohre vorgesehen sein, um mehr als ein Isolier- oder Reaktionsgefäß gleichzeitig bearbeiten zu können. Die Saug und Dispensiervorrichtung 8 erfüllt also die Funktion einer Pipette. Sog und Druck werden der Saug- und Dispensiervorrichtung 8 über Schläuche 9 vermittelt.

Zur Nukleinsäureisolierung können beispielsweise Reaktionsgefässe mit Zellen in den Rüttler/Halter 6 eingesetzt werden, in die mit der Dispensiervorrichtung 8 Lysepuffer eingefüllt wird. Nach Mischen werden die Zelllysate in Isoliergefässe überführt. Die Anordnung von Isoliergefässen wird daraufhin auf die Absaugvorrichtung 5 aufgesetzt und der Lysepuffer durch die Oberflächen in den Isoliergefässen durchgesaugt. Im Anschluß kann die Oberfläche mit einem Waschpuffer gespült werden, um Reste der Zelllysate zu entfernen, wobei auch der Waschpuffer nach unten abgesaugt wird. Schließlich wird Eluat in die Isoliergefässe dispensiert und nach eventuelllem nochmaligem Rütteln werden die abgelöste Nukleinsäuren nach oben entnommen und in Aufbewahrungsgefässe überführt.

Üblicherweise werden Wechselspitzen an der Saug- und Dispensiervorrichtung 8 verwendet, um eine Kontamination der Proben zu verhindern.

Die Fig. 2 bis 4 zeigen verschiedene Beispiele für geeignete Isoliergefässe zur Verwendung in der vorliegenden Erfindung.

In Fig. 2 ist ein trichterförmiges Isoliergefäß 10 mit einer Oberfläche 11, beispielsweise einer Membran, versehen, die auf einen Auffangbehälter 12 aufgesetzt ist, der ein schwammartiges Material 13 enthält, das der Aufnahme von Lysepuffer und Waschpuffer dient. In den Trichter wird Lysat oder eine sonstige Aufbereitung von Nukleinsäuren 14 gegeben. Das schwammartige Material 13 saugt die aufgetragene Flüssigkeit durch die Membran 11 hindurch. Vor der Zugabe des Eluierpuffer wird der Schwamm etwas von der Membran beabstandet, beispielsweise durch eine im Auffangbehälter 12 angeordnete Mechanik (nicht dargestellt). So wird beim letzten Schritt verhindert, daß der Eluatpuffer auch durch die Membran 11 gesaugt wird. Dieser verbleibt vielmehr auf der Oberfläche (Fig. 1b) und kann zusammen mit den Nukleinsäuren nach oben entnommen werden. Mit dieser Anordnung wird die Absaugvorrichtung 5 im Automaten nicht benötigt.

Fig. 3 zeigt ein weiteres Beispiel eines Isoliergefässes, daß über einen an seinem unteren Ende angeordneten Luer-Anschluß mittels eines Luer-Adapters 17 mit einem Aufffangbehälter 16 verbunden ist, der in diesem Fall keinen Schwamm enthält, sondern mittels eines Stutzen 18 mit einer Absaugvorrichtung verbunden ist. Lyse- und Waschpuffer können hier also durch Anlegen eines Vakuums durch die Membran 11 durchgesaugt werden. Beim Auftragen des Eluatpuffers bleibt das Vakuum abgeschaltet, so daß sich das Eluat nach oben entnehmen lässt. Durch die Verwendung eines Luer-Anschlusses sind individuelle Isoliergefässe der Anordnung von Isoliergefässen entnehmbar. Es versteht sich jedoch, daß der Vakuumauffangbehälter auch mit fest angebrachtetn Isoliergefässen kombinierbar ist.

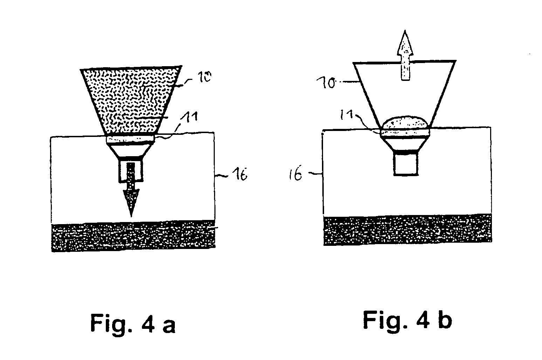

Fig. 4 zeigt schließlich eine Ausführungsform, bei der ein Auffangbehälter vorgesehen ist, in den die Puffer mittels Schwerkraft hineingesaugt werden. Der Eluatpuffer, der in geringem Volumen verwendet wird, hat kein hinreichendes Eigengewicht, um die Membran 11 zu durchdringen und kann wiederum nach oben abgenommen werden.

Die vorbeschriebene Erfindung wird durch die nachfolgenden Beispiele erläutert. Hierbei sind Beispiele 1 bis 17 im wesentlichen auf die Verwendung hydrophober Membranen gerichtet und Beispiele 1b bis 2b auf die Verwendung hydrophiler Membranen. Verschiedenartige, andere Ausgestaltungen der Verfahren werden für den Fachmann aus der vorstehenden Beschreibung und den Beispielen ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind.

### Beispiel 1

### Isolierung von Gesamt-RNA aus HeLa-Zellen

In einer Plastiksäule werden kommerziell erhältliche hydrophobe Nylon-Membranen (Beispielsweise ein Material der Fa. MSI: Magna SH mit einem Porendurchmesser von 1,2 µm oder ein Material der Fa. Pall GmbH: Hydrolon mit einem Porendurchmesser von 1,2 µm), die durch chemische Nachbehandlung hydrophobisiert wurden, einlagig eingebracht. Die Membranen werden auf einer Polypropylenfritte, die als mechanische Unterstützung dient, plaziert. Die Membranen werden in der Plastiksäule durch einen Spannring fixiert.

Die so vorbereitete Säule wird über eine Luerverbindung mit einer Vakuumkammer verbunden - alle Isolierungschritte werden mit Hilfe eines angelegten Vakuums durchgeführt.

Zur Isolierung werden 5x10⁵ HeLa-Zellen durch Zentrifugation pelletiert und der Überstand entfernt. Die Zellen werden durch Zugabe von 150 µl eines handelsüblichen Guanidinium-*iso-*thiocyanat-Puffer - wie z. B. RLT-Puffer der Fa. Qiagen - auf an sich aus dem Stand der Technik bekannte Weise lysiert. Dabei wird die Lyse durch mehrmaliges Auf- und Abpipettieren oder vortexen über einen Zeitraum von ca. 5 s unterstützt. Anschließend werden 150 µl 70 %-iges Ethanol zugefügt und durch Auf- und Abpipettieren oder durch vortexen über einen Zeitraum von ca. 5 s gemischt.

Das Lysat wird anschließend in die Plastiksäule pipettiert und durch Evakuierung der Vakuumkammer durch die Membran gesaugt. Unter den so eingestellten Bedingungen bleibt die RNA an der Membran gebunden. Anschließend wird mit einem ersten handelsüblichen Guanidinium-*iso*-thiocyanat-haltigen Waschpuffer - beispielsweise mit dem Puffer RW1 der Fa. Qiagen - und danach mit einem zweiten Tris-haltigen bzw. TRIS- und alkohol-haltigen Waschpuffer - z. B. mit dem Puffer RPE der Fa. Qiagen - gewaschen. Dabei werden die Waschpuffer jeweils durch Evakuierung der Vakuumkammer durch die Membran gesaugt. Nach dem letzten Waschschritt wird das Vakuum über einen Zeitraum von ca. 10 min aufrechterhalten, um die Membran zu trocknen. danach wird die Vakuumkammer belüftet.

Zur Elution werden 70 µl RNase-freies Wasser auf die Membran pipettiert, um die gereinigte RNA von der Membran abzulösen. Nach einer Inkubation über einen Zeitraum von 1 min bei einer Temperatur im Bereich von 10° bis 30°C wir das Eluat mittels einer Pipette von oben von der Membran abpipettiert und der Elutionsschritt wird zum Zweck einer vollständigen Elution noch einmal wiederholt.

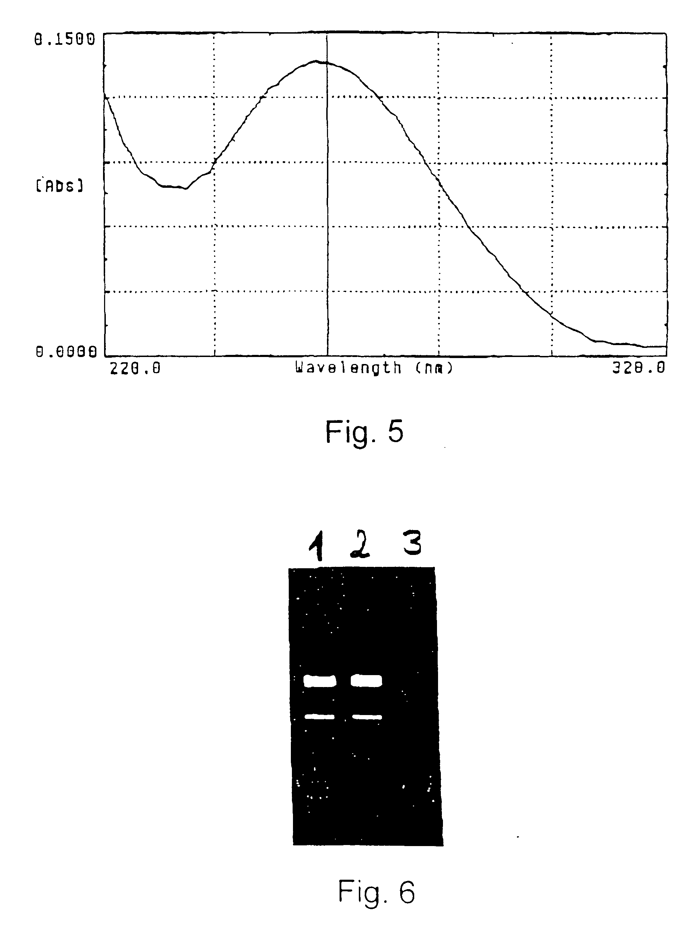

Die Menge an isolierter Gesamt-RNA wird anschließend durch photometrische Messung der Lichtabsorption bei einer Wellenlänge von 260 nm ermittelt. Die Qualität der so gewonnenen RNA wird durch die photometrische Bestimmung des Verhältnisses der Lichtabsorption bei 260 nm zu derjenigen bei 280 nm bestimmt (vgl. Fig. 5: Gesamt-RNA über Hydrolon 1.2 isoliert)

Die Ergebnisse der zwei Isolierungen mit hydrophoben Nylonmembranen (Nr. 1 und 2) sind in der nachfolgenden Tabelle 1 Vergleichsversuche, in denen zum einen hydrophiles Nylon (Nyaflo) (Nr. 3) sowie eine Silica-Membran eingesetzt wurde (Nr. 4), gegenübergestellt. Die dort wiedergegebenen Werte liefern einen überzeugenden Beleg für die beeindruckende Isolierungsleistung sowie Trennwirkung der erfindungsgemäß eingesetzten Materialien. Sie zeigen weiterhin, daß Silica-gel-Vliese eine deutlich geringe Ausbeute erbringen, was vermutlich auf ihre vlisartige Struktur und die damit verbundene Absorption des größten Teiles des Eluatpuffers zurückzuführen ist.

**Tabelle 1: RNA-Ausbeute und -Reinheit der nach Beispiel 1 isolierten Gesamt-RNA**

| Nr | Membrantypus | Ausbeute an Gesamt-RNA [µg] | E₂₆₀/E₂₈₀ |
|---|---|---|---|
| 1 | Magna SH 1,2 µm (hydrophobes Nylon) | 6,0 | 1.97 |
| 2 | Hydrolon 1,2 µm (hydrophobes Nylon) | 7,1 | 2.05 |
| 3 | Nyaflo (hydrophiles Nylon) | < 0.2 | nicht bestimmt |
| 4 | Hydrophile Silica-Membran | < 0,2 | nicht bestimmt |

Die isolierte RNA kann ferner auf Agarosegelen, die mit Ethidiumbromid angefärbt sind, analysiert werden. Hierzu werden beispielsweise 1,2 %-ige Formaldehyd-Agarose-Gele angefertigt. Das Ergebnis ist in Fig. 6 wiedergegeben.

In Fig. 6 verkörpert Spur 1 eine Gesamt-RNA, die über eine hydrophobe Nylon-Membran des Ursprungs Magna SH mit einem Porendurchmesser von 1,2 µm isoliert wurde.

Spur 2 stellt eine Gesamt-RNA dar, die über eine hydrophobe Nylon-Membran des Ursprungs Hydrolon mit einem Porendurchmesser von 1,2 µm isoliert wurde.

Spur 3 stellt das Chromatogramm einer Gesamt-RNA dar, die über eine Silica-Membran isoliert wurde.

Dabei wurden jeweils 50 µl der Gesamt-RNA Isolate analysiert. Fig. 6 liefert einen deutlichen Beleg dafür, daß unter Verwendung der Silica-Membran kein meßbarer Anteil an Gesamt-RNA isoliert werden kann.

### Beispiel 2

Isolierung freier RNA durch Bindung der RNA an hydrophobe Membranen mittels verschiedener Salz/Alkohol-Gemische. Bei diesem Beispiel werden Lysat und Waschlösungen durch Anlegen eines Vakuums über die hydrophobe Membran geführt.

In Plastiksäulen, die mit einer Vakuumkammer in Verbindung stehen, werden hydrophobe Nylon-Membranen (beispielsweise Hydrolon 1.2 µm der Fa. Pall) analog Beispiel 1 eingebracht.

100 µl einer Gesamt-RNA enthaltenden wässerigen Lösung werden jeweils mit 350 µl eines kommerziell erhältlichen Guanidinium-*iso*-thiocyanat enthaltenden Lysepuffers (beispielsweise Puffer RLT der Fa. Qiagen), 350 µl 1.2 M Natriumacetat-Lösung, 350 µl 2 M Natriumchlorid-Lösung, 350 µl 4 M Lithiumchlorid-Lösung durch Auf- und Abpipettieren gemischt.

Anschließend werden jeweils 250 µl Ethanol zugegeben und ebenfalls durch Auf- und Abpipettieren gemischt. Die RNAhaltigen Lösungen werden danach in die Plastiksäulen einpipettiert und durch Evakuierung der Vakuumkammer durch die Membran gesaugt. Unter den beschriebenen Bedingungen bleibt die RNA an den Membranen gebunden. Die Membranen werden anschließend - wie in Beispiel 1 beschrieben - gewaschen.

Abschließend wird die RNA - ebenfalls wie in Beispiel 1 beschrieben - von oben - von der Membran mittels einer Pipette entnommen.

Die Menge an isolierter Gesamt-RNA wirde durch photometrische Messung der Lichtabsorption bei 260 nm ermittelt. - Die Qualität der so gewonnenen RNA wird durch die photometrische Bestimmung der Verhältnisse der Lichtabsorption bei 260 nm zu der bei 280 nm bestimmt.

**Tabelle 2 : Isolierung freier RNA durch Bindung der RNA an hydrophobe Membranen mittels verschiedener Salz/Alkohol-Gemische**

| Nr | Salz/Alkohol-Gemisch | Ausbeute an Gesamt-RNA [µg] | E₂₆₀/E₂₈₀ |
|---|---|---|---|
| 1 | RLT-Puffer Qiagen / 35 %-iges Ethanol | 9.5 | 1.92 |
| 2 | 0.6 M Natriumacetat / 35 %-iges Ethanol | 8.5 | 1.98 |
| 3 | 1.0 M Natriumchlorid / 35 %-iges Ethanol | 7.9 | 1.90 |
| 4 | 2 M Lithiumchlorid / 35 %-iges Ethanol | 4.0 | 2.01 |

### Beispiel 3

### Isolierung von Gesamt-RNA aus HeLa-Zellen

In einer Plastiksäule werden kommerziell erhältliche hydrophobe Membranen, die aus verschiedenen Materialien bestehen, einlagig eingebracht. Die Membranen werden auf einer Polypropylenfritte, die als mechanische Unterstützung dient, plaziert und durch einen Spannring in der Plastiksäule fixiert. Die so vorbereitete Säule wird in ein Collection Tube gesetzt, die folgenden Isolierungsschritte werden mittels einer Zentrifugation durchgeführt.

Zur Isolierung werden 5x10⁵ HeLa-Zellen durch Zentrifugation pelletiert und der Überstand abgenomen. Die Zellen werden durch Zugabe von 150 µl eines handelsüblichen Guanidinium-Isothiocyanat-Puffers - wie z. B. RLT-Puffer der Fa. Qiagen - auf an sich aus dem Stand der Technik bekannte Weise lysiert. Dabei wird die Lyse durch mehrmaliges Auf- und Abpipettieren oder durch Vortexen über einen Zeitraum von 5 s unterstützt. Anschließend werde 150 µl 70 %-iges Ethanol zugefügt und durch mehrmaliges Auf- und Abpipettieren oder durch Vortexen über einen Zeitraum von 5 s gemischt.
Das Lysat wird anschließend in die Plastiksäule pipettiert und durch Zentrifugation bei 10000 x g für 1 min durch die Membran geführt. Anschließend wird mit einem handelsüblichen Guanidinium-Isothiocyanat-haltigen Waschpuffer - beispielsweise mit dem Puffer RW1 der Fa. Qiagen - und danach mit einem zweiten Tris- und alkoholhaltigen Waschpuffer - beispielsweise Puffer RPE der Fa. Qiagen - gewaschen. Dabei werden die Waschpuffer jeweils durch Zentrifugation durch die Membran geführt. Der letzte Waschschritt wird bei 20000 x g für 2 min durchgeführt, um die Membran zu trocknen.
Zur Elution werden 70 µl RNase-freies Wasser auf die Membran pipettiert, um die gereinigte RNA von der Membran zu lösen. Nach einer Inkubation von 1 - 2 min bei einer Temperatur im Bereich von 10 - 30 °C wird das Eluat mittels einer Pipette von oben von der Membran abpipettiert. Der Elutionsschritt wird einmal wiederholt, um eine vollständige Elution zu erreichen.
Die Menge an isolierter Gesamt-RNA wird anschließend durch photometrische Messung der Lichtabsorption bei einer Wellenlänge von 260 nm ermittelt. Die Qualität der RNA wird durch die photometrische Bestimmung des Verhältnisses der Lichtabsorption bei 260 nm zu derjenigen bei 280 nm bestimmt.
Die Ergebnisse der Isolierungen mit den verschiedenen hydrophoben Membranen sind in der nachfolgenden Tabelle 3 aufgeführt. Es werden 3 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 3: RNA-Ausbeute der nach Beispiel 3 isolierten Gesamt-RNA durch Bindung an hydrophobe Membranen**

| Hersteller | Membran | Material | RNA (µg) | 260 nm/ 280 nm |
|---|---|---|---|---|
| Pall Gelman Sciences | Hydrolon, 1,2 µm | hydrophobes Nylon | 6.53 | 1,7 |
| Pall Gelman Sciences | Hydrolon, 3 µm | hydrophobes Nylon | 9,79 | 1,72 |
| Pall Gelman Sciences | Fluoro Trans G | hydrophobes, caboxyliertes Polyvinylidendifluorid | 6,16 | 1,72 |
| Pall Gelman Sciences | NFWA | Acrylpolymer auf Nylongewebestützkörper | 2,91 | 1,73 |
| Pall Gelman Sciences | Hemasep V Medium | modifiziertes Polyester | 4,16 | 1,74 |
| Pall Gelman Sciences | Hemadyne | modifiziertes Polyester | 6,67 | 1,65 |
| Pall Gelman Sciences | V-800 R | leicht hydrophobes modifiziertes Acryl-Copolymer | 6,26 | 1,72 |
| Pall Gelman Sciences | Supor-450 PR | hydrophobes Polyethersulfon | 3,96 | 1,76 |
| Pall Gelman Sciences | Versapor - 1200R | leicht hydrophobes modifiziertes Acryl-Copolymer | 6,23 | 1,68 |
| Pall Gelman Sciences | Versapor - 3000R | leicht hydrophobes modifiziertes Acryl-Copolymer | 3,54 | 1,74 |
| Pall Gelman Sciences | Zefluor | Polytetrafluorethylen | 5,19 | 1,65 |
| Pall Gelman Sciences | Polypro - 450 | Polyesterfaser | 4,58 | 1,77 |
| GORE - TEX | Polypropylen-Lochfolie 93 | hydrophobes Polytetrafluorethylen | 3,6 | 1,59 |
| GORE - TEX | Polypropylen-Lochfolie 93 | hydrophobes Polytetrafluorethylen | 2,15 | 1,65 |
| GORE - TEX | Polypropylen-Lochfolie 93 | hydrophobes Polytetrafluorethylen | 1,59 | 1,72 |
| GORE - TEX | Polyester-Vlies 9316 | hydrophobes Polytetrafluorethylen | 3,61 | 1,69 |
| GORE - TEX | Polyprop.-Vlies 9317 | hydrophobes Polytetrafluorethylen | 2,87 | 1,70 |
| Millipore | Mitex Membrane | hydrophobes Polytetrafluorethylen | 1,98 | 1,62 |
| Millipore | DVHP | hydrophobes Polyvinylidenfluorid | 7,45 | 1,72 |
| MSI | Magna-SH, 1.2 µm | hydrophobes Nylon | 4,92 | 1,69 |
| MSI | Magna-SH, 5 µm | hydrophobes Nylon | 10,2 | 1,71 |
| MSI | Magna-SH, 10 µm | hydrophobes Nylon | 7,36 | 1,76 |
| MSI | Magna-SH, 20 µm | hydrophobes Nylon | 7,04 | 1,65 |

### Beispiel 4

### Isolierung freier RNA aus wäßriger Lösung

Entsprechend dem Beispiel 3 werden Plastiksäulen mit verschiedenen hydrophoben Membranen hergestellt.

100 µl einer Gesamt-RNA enthaltenden wäßrigen Lösung wird mit 350 µl eines kommerziell erhältlichen Guanidinium-Isothiocyanat enthaltenden Lysepuffers - z. B. RLT-Puffer der Fa. Qiagen - vermischt. Anschließend werden 250 µl Ethanol zugegeben und durch Auf- und Abpipettieren gemischt. Dieses Gemisch wird dann auf die Säule aufgetragen und durch Zentrifugation (10000 x g; 1 min) durch die Membran geführt. Die Membranen werden anschließend zweimal mit einem Puffer - z. B. RPE der Fa. Qiagen - gewaschen. Der Puffer wird jeweils durch Zentrifugation durch die Membranen geführt. Der letzte Waschschritt wird bei 20000 x g durchgeführt, um die Membranen zu trocknen.
Abschließend wird die RNA wie bereits im Beispiel 3 beschrieben mit RNase-freiem Wasser eluiert und mittels einer Pipette von der Membran abgenommen.
Die Menge an isolierter Gesamt-RNA wird anschließend durch photometrische Messung der Lichtabsorption bei einer Wellenlänge von 260 nm ermittelt und die Qualität der RNA durch die photometrische Bestimmung des Verhältnisses der Lichtabsorption bei 260 nm zu derjenigen bei 280 nm bestimmt.
Die Ergebnisse der Isolierungen mit den verschiedenen hydrophoben Membranen sind in der nachfolgenden Tabelle 4 aufgeführt. Es werden 3 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 4: Isolierung freier RNA aus wäßriger Lösung durch Bindung an hydrophobe Membranen**

| Hersteller | Membran | Material | RNA (µg | 260 nm/ 280 nm |
|---|---|---|---|---|
| Pall Gelman Sciences | Hydrolon, 1,2 µm | hydrophobes Nylon | 5,15 | 1,75 |
| Pall Gelman Sciences | Hydrolon, 3 µm | hydrophobes Nylon | 0,22 | 1,79 |
| Pall Gelman Sciences | Fluoro Trans G | hydrophobes, caboxyliertes Polyvinylidendifluorid | 5,83 | 1,79 |
| Pall Gelman Sciences | NFWA | Acrylpolymer auf Nylongewebestützkörper | 1,85 | 1,72 |
| Pall Gelman Sciences | Hemasep V Medium | modifiziertes Polyester | 4 | 1,79 |
| Pall Gelman Sciences | Hemadyne | modifiziertes Polyester | 0,47 | 2,1 |
| Pall Gelman Sciences | V-800 R | leicht hydrophobes modifiziertes Acryl-Copolymer | 2,74 | 1,77 |
| Pall Gelman Sciences | Supor-450 PR | hydrophobes Polyethersulfon | 5,97 | 1,71 |
| Pall Gelman Sciences | Zefluor | Polytetrafluorethylen | 8,67 | 1,69 |
| Pall Gelman Sciences | Polypro - 450 | Polyesterfaser | 5,09 | 1,78 |
| GORE - TEX | Polypropylen-Lochfolie 9337 | hydrophobes Polytetrafluorethylen | 5,96 | 1,62 |
| GORE - TEX | Polypropylen-Lochfolie 9336 | hydrophobes Polytetrafluorethylen | 7,43 | 1,71 |
| GORE - TEX | Polypropylen-Lochfolie 9335 | hydrophobes Polytetrafluorethylen | 4,35 | 1,63 |
| GORE - TEX | Polyester-Vlies 9316 | hydrophobes Polytetrafluorethylen | 5,92 | 1,67 |
| GORE - TEX | Polyprop.-Vlies 9317 | hydrophobes Polytetrafluorethylen | 8,7 | 1,66 |
| Millipore | Fluoropore PTFE | hydrophobes Polytetrafluorethylen | 8,46 | 1,70 |
| Millipore | DVHP | hydrophobes Polyvinylidenfluorid | 4,23 | 1,8 |
| MSI | Magna-SH, 1,2 µm | hydrophobes Nylon | 1,82 | 1,76 |

### Beispiel 5

### Isolierung von Gesamt-RNA aus HeLa-Zellen in Abhängigkeit von der Porengröße der Membran

Entsprechend dem Beispiel 3 werden Plastiksäulen mit verschiedenen hydrophoben Membranen unterschiedlicher Porengröße hergestellt.
Nach Beispiel 1 wird ein Zell-Lysat aus 5x10⁵ HeLa-Zellen hergestellt und über die Säulen geführt. Anschließend werden die Membran mit den kommerziell erhältlichen Puffern RW1 und RPE der Fa. Qiagen mittels Zentrifugation gewaschen. Der letzte Zentrifugationsschritt wird bei 20000 x g für 2 min durchgeführt, um die Membranen zu trocknen. Die Elution wird wie in Beispiel 1 beschrieben durchgeführt.
Die Ergebnisse sind in der nachfolgenden Tabelle 5 aufgeführt. Es werden 3 - 5 Parällelversuche pro Membran durchgeführt und jeweils der Mittelwert errechnet.

**Tabelle 5: RNA-Ausbeute der isolierten Gesamt-RNA durch Bindung an hydrophobe Membranen unterschiedlicher Porengröße**

| Hersteller | Membran | Material | Porengr. (µm) | RNA (µg) | 260 nm/ 280 hm |
|---|---|---|---|---|---|
| Infiltec | Polycon 0,01 | Hydrophiles Polycarbonat | 0,01 | 0,17 | 1,64 |
| Pall Gelman Sciences | Fluoro Trans G | hydrophobes, caboxyliertes Polyvinyl idendifluorid | 0,2 | 6,16 | 1,72 |
| Pall Gelman Sciences | Supor-450 PR | hydrophobes Polyethersulfon | 0,45 | 3,96 | 1,76 |
| Millipore | DVHP | hydrophobes Polyvinylidenfluorid | 0,65 | 7,45 | 1,72 |
| MSI | Magna-SH | hydrophobes Nylon | 1,2 | 4,92 | 1,69 |
| MSI | Magna-SH | hydrophobes Nylon | 5 | 10,2 | 1,71 |
| MSI | Magna-SH | hydrophobes Nylon | 10 | 7,36 | 1,76 |
| MSI | Magna-SH | hydrophobes Nylon | 20 | 7,04 | 1,65 |

### Beispiel 6

### Isolierung von genomischer DNA aus wäßriger Lösung

Entsprechend Beispiel 3 werden Plastiksäulen mit hydrophoben Membranen (beispielsweise Magna-SH, 5 µm der Fa. MSI) hergestellt. Die Durchführung der Aufreinigung wird mit handelsüblichen Puffern der Fa. Qiagen durchgeführt.
200 µl einer wäßrigen Lösung genomischer DNA aus Lebergewebe wird in PBS-Puffer hergestellt. Zu dieser Lösung werden 200 µl eines Guanidinium-Hydrochlorid-haltigen Puffers - z. B. AL der Firma Qiagen - gegeben und vermischt. Anschließend werden 210 µl Ethanol zugegeben und durch Vortexen vermischt. Das Gemisch wird entsprechend Beispiel 3 auf die Säule getragen und durch Zentrifugation durch die Membran geführt. Die Membran wird anschließend mit einem alkoholhaltigen Puffer - z. B. RW der Fa. Qiagen - gewaschen und getrocknet. Die Elution wird wie in Beispiel 3 beschrieben durchgeführt. Es werden drei Parallelversuche durchgeführt und der Mittelwert errechnet.
Die Menge an isolierter DNA wird anschließend durch photometrische Messung der Lichtabsorption bei einer Wellenlänge von 260 nm ermittelt und beträgt ca. 30 % der Ausgangsmenge. Das Verhältnis der Absorption bei 260 nm zu derjenigen bei 280 nm beträgt 1,82.

### Beispiel 7

### Isolierung von genomischer DNA aus Gewebe

Entsprechend Beispiel 3 werden Plastiksäulen mit hydrophoben Membranen (beispielsweise Magna-SH, 5 µm der Fa. MSI) hergestellt. Die Durchführung der Aufreinigung wird mit handelsüblichen Puffern der Fa. Qiagen durchgeführt.
10 mg Nierengewebe (Maus) wird mit 180 µl ATL versetzt und durch einen mechanischen Homogenisator zermahlen. Anschließend wird Proteinase K (ca. 0,4 mg gelöst in 20 µl Wasser) zum Ansatz gegeben und bei 55 °C für 10 min inkubiert. Nach Zusatz von 200 µl eines Guanidinium-Hydrochlorid-haltigen Puffers - z. B. AL der Firma Qiagen - Mischen und einer 10 minütigen Inkubation bei 70 °C wird 200 µl Ethanol unter den Ansatz gemischt. Dieses Gemisch wird auf die Säule getragen und durch Zentrifugation über die Membran geführt. Die Membran wird mit einem alkoholhaltigen Puffer - z. B. RW der Fa. Qiagengewaschen und anschließend durch Zentrifugation getrocknet. Die Elution wird wie in Beispiel 3 beschrieben durchgeführt. Es werden drei Parallelversuche durchgeführt und der Mittelwert errechnet.
Die Menge an isolierter DNA wird anschließend durch photometrische Messung der Lichtabsorption bei einer Wellenlänge von 260 nm ermittelt und beträgt im Durchschnitt 9,77 µg. Das Verhältnis der Absorption bei 260/280 nm beträgt 1,74.

### Beispiel 8

### Immobilisierung von Gesamt-RNA aus wäßriger Lösung mittels unterschiedlicher chaotroper Agenzien

Entsprechend Beispiel 3 werden Plastiksäulen mit hydrophoben Membranen hergestellt.
100 µl einer Gesamt-RNA enthaltenden wäßrigen Lösung wird mit 350 µl eines Lysepuffers, der Guanidinium-Isothiocyanat bzw. Guanidinium-Hydrochlorid in unterschiedlichen Konzentrationen enthält, vermischt. Anschließend werden 250 µl Ethanol zugegeben und durch Auf- und Abpipettieren gemischt. Dieses Gemisch wird dann auf die Säule aufgetragen und durch Zentrifugation (10000 x g; 1 min) durch die Membran geführt. Die Membranen werden anschließend zweimal mit einem alkoholhaltigen Puffer - z. B. RPE der Fa. Qiagen - gewaschen. Der Puffer wird jeweils durch Zentrifugation durch die Membranen geführt. Der letzte Waschschritt wird bei 20000 x g durchgeführt, um die Membranen zu trocknen. Die Elution wird wie in Beispiel 3 durchgeführt. Es werden Doppelbestimmungen durchgeführt und jeweils der Mittelwert angegeben.
Die Ergebnisse sind in Tabelle 6 aufgeführt.

**Tabelle 6: RNA-Ausbeute aus wäßriger Lösung mittels chaotroper Agenzien**

| Membran | chaotropes Agens und Konzentration im Bindeansatz | Ausbeute an Gesamt-RNA (µg |
|---|---|---|
| Hydrolon, 1,2 µm | GITC, 500 mM | 2,3 |
| Hydrolon, 1,2 µm | GITC, 1 M | 0,8 |
| Hydrolon, 1,2 µm | GITC, 3 M | 0,9 |
| Fluoro Trans G | GITC, 500 mM | 0,4 |
| Fluoro Trans G | GITC, 1 M | 1,25 |
| Fluoro Trans G | GITC, 3 M | 0,6 |
| Hydrolon, 1,2 µm | GuHCl, 500 mM | 2,6 |
| Hydrolon, 1,2 µm | GuHCl, 1 M | 6,7 |
| Hydrolon, 1,2 µm | GuHCl, 3 M | 2,9 |
| Fluoro Trans G | GuHCl, 500 mM | 0,4 |
| Fluoro Trans G | GuHCl, 1 M | 1,25 |
| Fluoro Trans G | GuHCl, 3 M | 0,6 |

### Beispiel 9

### Immobilisierung von Gesamt-RNA aus wäßriger Lösung mittels unterschiedlicher Alkohole

Entsprechend Beispiel 3 werden Plastiksäulen mit hydrophoben Membranen-hergestellt.
100 µl einer Gesamt-RNA enthaltenden wäßrigen Lösung wird mit 350 µl eines Guanidinium-Isothiocyanat enthaltenden Lysepuffers (Konzentration 4 M) vermischt. Anschließend werden unterschiedliche Mengen an Ethanol bzw. Isopropanol zugegeben und mit RNase-freiem Wasser auf 700 µl aufgefüllt und gemischt. Dieses Gemisch wird dann auf die Säule aufgetragen und entsprechend Beispiel 4 durch die Membran geführt und gewaschen. Die Elution erfolgte ebenfalls wie-in Beispiel 3. Es werden Doppelbestimmungen durchgeführt und jeweils der Mittelwert angegeben.

Die Ergebnisse sind in Tabelle 7 aufgeführt.

**Tabelle 7: RNA-Ausbeute aus wäßriger Lösung mit unterschiedlichen Alkoholen im Bindeansatz**

| Membran | Alkohol und Konzentration im Bindeans | Ausbeute an Gesamt-RNA (µg |
|---|---|---|
| Hydrolon, 1,2 µm | Ethanol, 5 % | 0,7 |
| Hydrolon, 1,2 µm | Ethanol, 30 % | 2,85 |
| Hydrolon, 1,2 µm | Ethanol, 50 % | 4,5 |
| DVHP | Ethanol, 5 % | 0,4 |
| DVHP | Ethanol, 30 % | 1,25 |
| DVHP | Ethanol, 50 % | 0,6 |
| Hydrolon, 1,2 µm | Isopropanol, 5 % | 0,35 |
| Hydrolon, 1,2 µm | Isopropanol, 30 % | 4,35 |
| Hydrolon, 1,2 µm | Isopropanol, 50 % | 3,2 |
| DVHP | Isopropanol, 10 % | 1,35 |
| DVHP | Isopropanol, 30 % | 4,1 |
| DVHP | Isopropanol, 50 % | 3,5 |

### Beispiel 10

### Immobilisierung von Gesamt-RNA aus wäßriger Lösung mit unterschiedlichen pH-Werten

Entsprechend Beispiel 3 werden Plastiksäulen mit hydrophoben Membranen hergestellt.
100 µl einer Gesamt-RNA enthaltenden wäßrigen Lösung wird mit 350 µl eines Guanidinium-Isothiocyanat enthaltenden Lysepuffers (Konzentration 4 M) vermischt. Der Puffer enthält 25 mM Natrium-Citrat und wird auf unterschiedliche pH-Werte mittels HCl bzw. NaOH eingestellt. Anschließend wird 250 µl Ethanol zugegeben und gemischt. Dieses Gemisch wird dann auf die Säule aufgetragen und entsprechend Beispiel 4 durch die Membran geführt und gewaschen. Die Elution erfolgte ebenfalls wie in Beispiel 3. Es werden Doppelbestimmungen durchgeführt und jeweils der Mittelwert angegeben.
Die Ergebnisse sind in Tabelle 8 aufgeführt.

**Tabelle 8: RNA-Ausbeute aus wäßriger Lösung mit unterschiedlichen pH-Werten im Bindeansatz**

| Membran | pH-Wert im Bindeansatz | Ausbeute an Gesamt-RNA (µg |
|---|---|---|
| Hydrolon, 1,2 µm | pH 3 | 0,15 |
| Hydrolon, 1,2 µm | pH 9 | 1,6 |
| Hydrolon, 1,2 µm | pH 11 | 0,05 |
| Fluoro Trans G | pH 1 | 0,45 |
| Fluoro Trans G | pH 9 | 2,85 |
| Fluoro Trans G | pH 11 | 0,25 |

### Beispiel 11

### Immobilisierung von Gesamt-RNA aus wäßriger Lösung mittels unterschiedlicher Salze

Entsprechend Beispiel 3 werden Plastiksäulen mit hydrophoben Membranen hergestellt.
100 µl einer Gesamt-RNA enthaltenden wäßrigen Lösung wird mit 350 µl eines salzhaltigen Lysepuffers (NaCl, KCl, MgSO₄) vermischt. Anschließend wird 250 µl H₂O oder Ethanol zugegeben und gemischt. Dieses Gemisch wird dann auf die Säule aufgetragen und entsprechend Beispiel 4 durch die Membran geführt, gewaschen und eluiert. Es werden Doppelbestimmungen durchgeführt und jeweils der Mittelwert angegeben.
Die Ergebnisse sind in Tabelle 9 aufgeführt.

**Tabelle 9: RNA-Ausbeute aus wäßriger Lösung mit verschiedenen Salzen im Bindeansatz**

| Membran | Salz-Konzentration im Bindeansatz | Ausbeute an Gesamt-RNA (µg |
|---|---|---|
| Hydrolon, 1,2 µm | NaCl, 100 mM; ohne Ethanol | 0,1 |
| Hydrolon, 1.2 µm | NaCl, 1 M; ohne Ethanol | 0,15 |
| Hydrolon, 1,2 µm | NaCl, M; ohne Ethanol | 0,3 |
| Hydrolon, 1,2 µm | KCl, 10 mM; ohne Ethanol | 0,2 |
| Hydrolon, 1,2 µm | KCl, 1 M; ohne Ethanol | 0,1 |
| Hydrolon, 1,2 µm | KCl, 3 M; ohne Ethanol | 0,25 |
| Hydrolon, 1,2 µm | MgSO₄, 100 mM; ohne Ethanol | 0,05 |
| Hydrolon, 1,2 µm | MSO₄, 750 mM; ohne Ethanol | 0,15 |
| Hydrolon, 1,2 µm | MgSO₄, 2 M; ohne Ethanol | 0,48 |
| Hydrolon, 1,2 µm | NaCl, 500 mM; mit Ethanol | 2,1 |
| Hydrolon, 1,2 µm | NaCl, 1 M; mit Ethanol | 1,55 |
| Hydrolon, 1,2 µm | NaCl, 2.5 M; mit Ethanol | 1,35 |
| Hydrolon, 1,2 µm | KCl, 500 mM; mit Ethanol | 1,6 |
| Hydrolon, 1,2 µm | KCl, 1 M; mit Ethanol | 2,1 |
| Hydrolon, 1,2 µm | KCl, 1.5 M; mit Ethanol | 3,5 |
| Hydrolon, 1,2 µm | MgSO₄, 10 mM; mit Ethanol | 1,9 |
| Hydrolon, 1,2 µm | MgSO₄, 100 mM; mit Ethanol | 4,6 |
| Hydrolon, 1,2 µm | MgSO₄, 500 M; mit Ethanol | 2 |

### Beispiel 12

### Immobilisierung von Gesamt-RNA aus wäßriger Lösung mittels unterschiedlicher Pufferbedingungen

Entsprechend Beispiel 3 werden Plastiksäulen mit hydrophoben Membranen hergestellt.
100 µl einer Gesamt-RNA enthaltenden wäßrigen Lösung wird mit 350 µl eines Guanidinium-Isothiocyanat enthaltenden Lysepuffers (Konzentration 2,5 M) vermischt. Der Lysepuffer wird mit verschiedenen Konzentrationen an Natrium-Citrat, pH 7, bzw. Natrium-Oxalat versetzt. Anschließend wird 250 µl Ethanol zugegeben und gemischt. Dieses Gemisch wird dann auf die Säule aufgetragen und entsprechend Beispiel 4 durch die Membran geführt, gewaschen und eluiert.
Die Ergebnisse sind in Tabelle 10 aufgeführt. Es werden Doppelbestimmungen durchgeführt und jeweils der Mittelwert angegeben.

**Tabelle 10: RNA-Ausbeute aus wäßriger Lösung mit verschiedenen Pufferkonzentrationen im Bindeansatz**

| Membran | Natrium-Citrat im Lysepuffer | Ausbeute an Gesamt-RNA (µg |
|---|---|---|
| Hydrolon, 1,2 µm | Na-Citrat, 10 mM | 2,2 |
| Hydrolon, 1,2 µm | Na-Citrat, 100 mM | 2,4 |
| Hydrolon, 1,2 µm | Na-Citrat, 500 mM | 3,55 |
| Supor-450 PR | Na-Citrat, 10 mM | 1,1 |
| Supor-450 PR | Na-Citrat, 100 mM | 1,15 |
| Supor-450 PR | Na-Citrat, 500 mM | 0,2 |
| Hydrolon 1,2 µm | Na-Oxalat, 1 mM | 1,5 |
| Hydrolon 1,2 µm | Na-Oxalat, 25 mM | 1,05 |
| Hydrolon 1,2 µm | Na-Oxalat, 50 mM | 0,9 |
| Supor-450 PR | Na-Oxalat, 1 mM | 1,9 |
| Supor-450 PR | Na-Oxalat, 25 mM | 1,3 |
| Supor-450 PR | Na-Oxalat, 50 mM | 1,7 |

### Beispiel 13

### Immobilisierung von Gesamt-RNA aus wäßriger Lösung durch Phenol

Entsprechend Beispiel 3 werden Plastiksäulen mit hydrophoben Membranen (beispielsweise Hydrolon, 1,2 µm der Fa. Pall Gelman Sciences) hergestellt.
Wäßrige RNA-Lösung wird mit 700 µl Phenol vermischt und über die Membranen mittels Zentrifugation geführt. Entsprechend Beispiel 4 werden die Membranen gewaschen und die RNA eluiert. Es werden Doppelbestimmungen durchgeführt und jeweils der Mittelwert angegeben.
Die Menge an isolierter RNA wird anschließend durch photometrische Messung der Lichtabsorption bei einer Wellenlänge von 260 nm ermittelt und beträgt im Durchschnitt 10,95 µg. Das Verhältnis der Absorption bei 260/280 nm beträgt 0,975.

### Beispiel 14

### Waschen der immobilisierten Gesamt-RNA unter verschiedenen Salzkonzentrationen

Entsprechend Beispiel 3 werden Plastiksäulen mit hydrophoben Membranen hergestellt.
100 µl einer Gesamt-RNA enthaltenden wäßrigen Lösung wird mit 350 µl eines Guanidinium-Isothiocyanat enthaltenden Lysepuffers (Konzentration 4 M) vermischt. Anschließend wird 250 µl Ethanol zugegeben und gemischt. Dieses Gemisch wird dann auf die Säule aufgetragen und entsprechend Beispiel 4 durch die Membran geführt. Die Membranen werden anschließend zweimal mit einem Puffer, der unterschiedliche Konzentrationen an NaCl und 80 % Ethanol enthält, gewaschen. Der Puffer wird jeweils durch Zentrifugation durch die Membranen geführt. Der letzte Waschschritt wird bei 20000 x g durchgeführt, um die Membranen zu trocknen. Die Elution erfolgt ebenfalls wie in Beispiel 3. Es werden Doppelbestimmungen durchgeführt und jeweils der Mittelwert angegeben.
Die Ergebnisse sind in Tabelle 11 aufgeführt.

**Tabelle 11: RNA-Ausbeute aus wäßriger Lösung mit NaCl im Waschpuffer**

| Membran | NaCl im Waschpuffer | Ausbeute an Gesamt-RNA (µg |
|---|---|---|
| Hydrolon, 1,2 µm | NaCl, 10 mM | 1,4 |
| Hydrolon, 1,2 µm | NaCl, 50 mM | 3,15 |
| Hydrolon, 1,2 µm | NaCl, 100 mM | 3 |
| DVHP | NaCl, 10 mM | 2,7 |
| DVHP | NaCl. 50 mM | 2,85 |
| DVHP | NaCl. 100 mM | 2,7 |

### Beispiel 15

### Elution der immobilisierten Gesamt-RNA unter verschiedenen Salz- und Pufferbedingungen

Entsprechend Beispiel 3 werden Plastiksäulen mit hydrophoben Membranen hergestellt.
100 µl einer Gesamt-RNA enthaltenden wäßrigen Lösung wird mit 350 µl eines Guanidinium-Isothiocyanat enthaltenden Lysepuffers (Konzentration 4 M) vermischt. Anschließend wird 250 µl Ethanol zugegeben und gemischt. Dieses Gemisch wird dann auf die Säule aufgetragen und entsprechend Beispiel 3 durch die Membran geführt und gewaschen.
Zur Elution werden 70 µl einer NaCl-haltigen Lösung, eines Tris/HCl-Puffers (pH 7) oder einer Natrium-Oxalat Lösung (pH 7,2) auf die Membran pipettiert, um die gereinigte RNA von der Membran zu lösen. Nach einer Inkubation von 1 - 2 min bei einer Temperatur im Bereich von 10 - 30 °C wird das Eluat mittels einer Pipette von oben von der Membran abpipettiert. Der Elutionsschritt wird einmal wiederholt, um eine vollständige Elution zu erreichen. Es werden Doppelbestimmungen durchgeführt und jeweils der Mittelwert angegeben.
Die Ergebnisse sind in Tabelle 12 zusammengefaßt.

**Tabelle 12: RNA-Ausbeute aus wäßriger Lösung mit NaCl bzw. Tris/HCl im Elutionspuffer**

| Membran | NaCl bzw. Tris im Elutionspuffer | Ausbeute an Gesamt-RNA (µg) |
|---|---|---|
| Hydrolon, 1,2 µm | NaCl, 1 mM | 1,35 |
| Hydrolon, 1,2 µm | NaCl, 50 mM | 1,2 |
| Hydrolon, 1,2 µm | NaCl, 250 mM | 0,45 |
| DVHP | NaCl, 1 mM | 0,9 |
| DVHP | NaCl, 50 mM | 0,35 |
| DVHP | NaCl, 500 mM | 0,15 |
| Hydrolon, 1,2 µm | Tris, 1 mM | 0,35 |
| Hydrolon, 1,2 µm | Tris, 10 mM | 0,75 |
| DVHP | Tris, 1 mM | 1,5 |
| DVHP | Tris, 50 mM | 1 |
| DVHP | Tris, 250 mM | 0,1 |
| Hydrolon 1,2 µm | Na-Oxalat, 1 mM | 0,45 |
| Hydrolon 1,2 µm | Na-Oxalat, 10 mM | 0,65 |
| Hydrolon 1,2 µm | Na-Oxalat, 50 mM | 0,3 |
| DVHP | Na-Oxalat, 1 mM | 2 |
| DVHP | Na-Oxalat, 10 mM | 1,55 |
| DVHP | Na-Oxalat, 50 mM | 0,15 |

### Beispiel 16

### Einsatz von Gesamt-RNA in einer "Real Time" Quantitativen RT-PCR unter Verwendung des 5'-Nuklease PCR-Assays zur Amplifikation und Detektion von β-Aktin mRNA

Entsprechend dem Beispiel 3 werden Plastiksäulen mit einer kommerziell erhältlichen Membran (Pall-Gelman Sciences, Hydrolon 1,2 µm) hergestellt. Zur Isolierung von RNA werden 1 x 10⁵ HeLa-Zellen eingesetzt und die Aufreinigung der Gesamt-RNA wird wie in Beispiel 3 beschrieben durchgeführt. Die Elution erfolgt mit 2 x 60 µl H₂O wie in Beispiel 3 beschrieben. Zur vollständigen Entfernung restlicher geringer Mengen an DNA wird die Probe mit einer DNase vor der Analyse behandelt. Es wird eine "Ein-Gefäß 'Real Time' Quantitative RT-PCR" unter Verwendung des kommerziell erhältlichen Reagenzsystems von Perkin-Elmer (TaqMan^{(™)} PCR Reagent Kit) unter Einsatz einer M-MLV Reversen Transkriptase durchgeführt. Durch den Einsatz spezifischer Primer und einer spezifischen TaqMan-Sonde für β-Aktin (TaqMan^{(™)} β-Actin Detection Kit der Firma Perkin Elmer) werden die β-Aktin mRNA-Moleküle in der Gesamt-RNA Probe zunächst in β-Aktin cDNA umgeschrieben und anschließend direkt ohne Unterbrechung der Gesamtreaktion in dem gleichen Reaktionsgefäß amplifiziert und detektiert. Die Reaktionsansätze werden entsprechend den Anweisungen des Herstellers hergestellt. Es werden drei verschiedene Mengen an der isolierten Gesamt-RNA verwendet (1, 2, 4 µl Eluat) und eine dreifache Bestimmung durchgeführt. Als Kontrolle werden drei Ansätze ohne RNA mitgeführt. Die cDNA Synthese findet für 1 Stunde bei 37 °C statt, direkt gefolgt von einer PCR, die 40 Zyklen umfaßt. Die Reaktionen und die Analysen werden auf einem ABI PRISM^{(™)} 7700 Sequence Detector der Firma Perkin Elmer Applied Biosystems durchgeführt. Jedes während eines PCR-Zyklus entstehende Amplikon erzeugt ein lichtemmitierendes Molekül, das durch Abspaltung von der TaqMan-Sonde entsteht. Damit ist das insgesamt entstehende Lichtsignal direkt proportional zur entstehenden Amplikonmenge und damit zur ursprünglich in der Gesamt-RNA Probe vorhandenen Transkriptmenge. Das emmitierte Licht wird von dem Gerät gemessen und durch ein Computerprogramm ausgewertet. Der PCR-Zyklus, bei dem das Lichtsignal das erste Mal sicher oberhalb des Hintergrundrauschens detektiert wird, wird als "Threshold Cycle" (ct) bezeichnet. Dieser Wert ist ein Maß für die in der Probe vorhandene Menge an der spezifisch amplifizierter RNA. Für die eingesetzte Menge von 1 µl an mit dem hier beschriebenen Verfahren isolierter Gesamt-RNA ergibt sich ein durchschnittlicher ct-Wert von 17,1, für 2 µl an Gesamt-RNA ein ct-Wert von 16,4 und für 4 µl an Gesamt-RNA ein ct-Wert von 15,3. Hieraus ergibt sich ein linearer Zusammenhang von eingesetzter Gesamt-RNA und dem ct-Wert. Dies zeigt, daß die Gesamt-RNA frei Substanzen ist, die die Amplifikationsreaktion hemmen könnten. Die Kontrollansätze, die keine RNA enthalten, erzeugen keine Signale.

### Beispiel 17

### Einsatz von Gesamt-RNA in einer RT-PCR zur Amplifikation und Detektion von β-Aktin mRNA.

Entsprechend dem Beispiel 3 werden Plastiksäulen mit einer kommerziell erhältlichen Membran (Fa. Pall Gelman Sciences, Hydrolon der Porengröße 1,2 bzw. 3 µm; Fa. Sartorius, Sartolon der Porengröße 0,45 µm) hergestellt.
Zur Isolierung von RNA werden zwei verschiedene Ausgangsmaterialien eingesetzt;
1) Gesamt-RNA aus Leber (Maus) in wäßriger Lösung, Aufreinigung und Elution erfolgt wie in Beispiel 4 beschrieben und
2) 5 X 10⁵ HeLa-Zellen, die Aufreinigung der Gesamt-RNA sowie die Elution wird wie in Beispiel 3 beschrieben durchgeführt.

Es werden jeweils 20 ng der isolierten Gesamt-RNA eingesetzt. Als Kontrolle wird RNA, die mittels des RNeasy-Kits (Fa. Qiagen) aufgereinigt wurde, und ein Ansatz ohne RNA mitgeführt.

Es wird eine RT-PCR unter Standardbedingungen mit diesen Proben durchgeführt. Für die Amplifikation werden zwei verschiedene Primerpaare für das β-Aktin verwendet. Ein 150 Bp großes Fragment dient als Nachweis der Sensitivität, ein 1,7 kBp großes Fragment dient der Integrität der RNA. Aus der RT-Reaktion wird 1 µl entnommen und in der anschließenden PCR eingesetzt. Es werden für das kleine Fragment 25 Zyklen, für das große Fragment 27 Zyklen durchgeführt. Die Anlagerungstemperatur beträgt 55°C. Die Amplifikate werden anschließend auf einem nicht denaturierenden Gel aufgetragen und analysiert.

Für die eingesetzte Menge von 20 ng an mit dem hier beschriebenen Verfahren isolierter Gesamt-RNA lassen sich in der RT-PCR die entsprechenden DNA-Fragmente nachweisen. Bei Verwendung von Gesamt-RNA aus Mausleber läßt sich kein Transkript nachweisen, die hier verwendeten Bedingungen sind auf humanes β-Aktin angepaßt. Die Kontrollansätze, die keine RNA enthalten, erzeugen keine Signale.

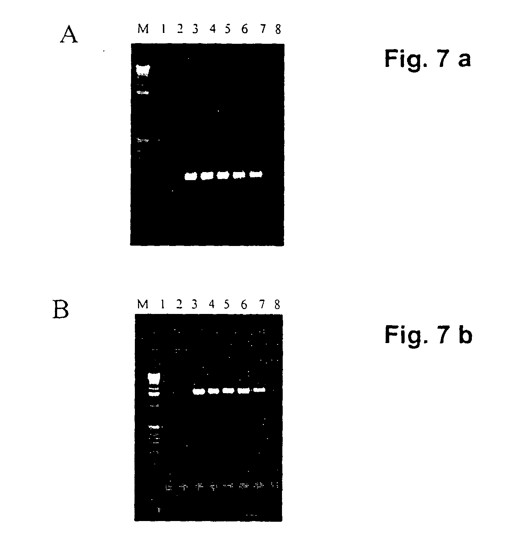

Fig. 7 zeigt Ethidiumbromid-gefärbte Gele einer elektrophoretischen Auftrennung der RT-Reaktionen.
A: Spur 1 bis 8: RT-PCR eines 150 Bp-Fragmentes; Spur 1, 2: RNA aus wäßriger Lösung mit der Membran.Hydrolon 1,2 µm aufgereinigt; Spur 3, 4: RNA aus HeLa-Zellen mit der Membran Sartolon aufgereinigt; Spur 5, 6: RNA aus HeLa-Zellen mit der Membran Hydrolon 3 µm aufgereinigt; Spur 7: RNA aufgereinigt mittels RNeasy-Mini-Kit; Spur 8: Kontrolle ohne RNA.
B: Spur 1 bis 8: RT-PCR eines 1,7 kBp-Fragmentes; Spur 1, 2: RNA aus wäßriger Lösung mit der Membran Hydrolon 1,2 µm aufgereinigt; Spur 3, 4: RNA aus HeLa-Zellen mit der Membran Sartolon aufgereinigt; Spur 5, 6: RNA aus HeLa-Zellen mit der Membran Hydrolon 3 µm aufgereinigt; Spur 7: RNA aufgereinigt mittels RNeasy-Mini-Kit; Spur 8: Kontrolle ohne RNA.

### Beispiel 1b

### Isolierung von Gesamt-RNA aus HeLa-Zellen durch Bindung an hydrophile Membranen

In einer Plastiksäule werden kommerziell erhältliche hydrophile Membranen, die aus verschiedenen Materialien bestehen, einlagig eingebracht. Entsprechend Beispiel 3 werden die Membranen auf einer Polypropylenfritte plaziert und durch einen Spannring fixiert.
Zur Isolierung werden 5x10⁵ HeLa-Zellen eingesetzt. Die folgenden Isolierungsschritte und die Elution der Nucleinsäure werden wie in Beispiel 3 beschrieben durchgeführt.
Die Menge an isolierter Gesamt-RNA wird anschließend durch photometrische Messung der Lichtabsorption bei einer Wellenlänge von 260 nm ermittelt. Die Qualität der RNA wird durch die photometrische Bestimmung des Verhältnisses der Lichtabsorption bei 260 nm zu derjenigen bei 280 nm bestimmt.
Die Ergebnisse der Isolierungen mit den verschiedenen hydrophilen Membranen sind in der nachfolgenden Tabelle 1b aufgeführt. Es werden 2 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 1b: RNA-Ausbeute der nach Beispiel 1b isolierten Gesamt-RNA durch Bindung an hydrophile Membranen**

| Hersteller | Membran | Material | RNA (µg) | 260 nm/ 280nm |
|---|---|---|---|---|
| Pall Gelman Sciences | I.C.E.-450 | hydrophiles Polyethersulfon | 6,36 | 1,8 |
| Pall Gelman Sciences | I.C.E.-450sup | hydrophiles Polyethersulfon auf einem Polyestergewebe | 3,07 | 1,71 |
| Pall Gelman Sciences | Premium Release | hydrophile Polyestermembran | 1,66 | 1,63 |
| Pall Gelman Sciences | Supor - 800 | hydrophiles Polyethersulfon | 4,12 | 1,7 |
| Pall Gelman Sciences | Supor - 450 | hydrophiles Polyethersulfon | 4,69 | 1,69 |
| Pall Gelman Sciences | Supor - 100 | hydrophiles Polyethersulfon | 3,25 | 1,71 |
| GORE - TEX | Polypropylen 9339 | hydrophiles Polytetrafluorethylen auf Polypropylen Gewebe | 1,08 | 1,65 |
| GORE - TEX | Polypropylen-Vlies 9338 | hydrophiles Polytetrafluorethylen auf Polypropylen-Vlies | 3,97 | 1,67 |
| GORE - TEX | Polyester-Vlies 9318 | hydrophiles Polytetrafluorethylen auf Polypropylen-Vlies | 3,61 | 1,69 |
| Millipore | Durapore PVDF | hydrophilisiertes Polyvinylidenfluorid | 5,6 | 1,69 |
| Millipore | hydrophylisierte PTFE | hydrophilisiertes Polytetrafluorethylen | 3,14 | 1,66 |
| Millipore | Durapore PVDF | hydrophilisiertes Polyvinylidenfluorid | 3,12 | 1,63 |
| Sartorius | Membranfilter Typ 250 | hydrophiles Polyamid | 4,3 | 1,66 |
| Infiltec | Polycon 0,01 | hydrophiles Polycarbonat | 0,17 | 1,64 |
| Infiltec | Polycon 0,1 | hydrophiles Polycarbonat | 0,73 | 1,68 |
| Infiltec | Polycon 1 | hydrophiles Polycarbonat | 3,33 | 1,86 |

### Beispiel 2b

### Isolierung freier RNA aus wäßriger Lösung durch Bindung an hydrophile Membranen

Entsprechend dem Beispiel 1b werden Plastiksäulen mit verschiedenen hydrophilen Membranen hergestellt.
100 µl einer Gesamt-RNA enthaltenden wäßrigen Lösung wird mit 350 µl eines kommerziell erhältlichen Guanidinium-Isothiocyanat enthaltenden Lysepuffers - z. B. RLT-Puffer der Fa. Qiagen - vermischt. Anschließend werden 250 µl Ethanol zugegeben und durch Auf- und Abpipettieren gemischt. Dieses Gemisch wird dann auf die Säule aufgetragen und entsprechend Beispiel 4 durch die Membran geführt, gewaschen und getrocknet.
Abschließend wird die RNA wie bereits im Beispiel 3 beschrieben mit RNase-freiem Wasser eluiert und mittels einer Pipette von der Membran abgenommen.
Die Menge an isolierter Gesamt-RNA wird anschließend durch photometrische Messung der Lichtabsorption bei einer Wellenlänge von 260 nm ermittelt und die Qualität der RNA durch die photometrische Bestimmung des Verhältnisses der Lichtabsorption bei 260 nm zu derjenigen bei 280 nm bestimmt.
Die Ergebnisse der Isolierungen mit den verschiedenen hydrophilen Membranen sind in der nachfolgenden Tabelle 2b aufgeführt. Es werden 2 - 5 Parallelversuche pro Membran durchgeführt und jeweils der Mittelwert errechnet. Durch die Verwendung einer Silica-Membran kann kein meßbarer Anteil an Gesamt-RNA isoliert werden, wenn das Eluat durch eine Abnahme von oben von der Membran gewonnen wird.

**Tabelle 2b: Isolierung freier RNA aus wäßriger Lösung durch Bindung an hydrophile Membranen**

| Hersteller | Membran | Material | RNA (µg) | 260 nm/ 280nm |
|---|---|---|---|---|
| Pall Gelman Sciences | I.C.E.-450 | hydrophiles Polyethersulfon | 1,92 | 1,82 |
| Pall Gelman Sciences | I.C.E.-450sup | hydrophiles Polyethersulfon auf einem Polyestergewebe | 0,87 | 1,67 |
| Pall Gelman Sciences | Supor - 800 | hydrophiles Polyethersulfon | 3,93 | 1,74 |
| Pall Gelman Sciences | Supor - 450 | hydrophiles Polyethersulfon | 1,78 | 1,74 |
| Pall Gelman Sciences | Supor - 100 | hydrophiles Polyethersulfon | 1,04 | 1,68 |
| GORE - TEX | Polypropylen 9339 | hydrophiles Polytetrafluorethylen auf Polypropylen Gewebe | 0,43 | 1,48 |
| GORE - TEX | Polypropylen-Vlies 9338 | hydrophiles Polytetrafluorethylen auf Polypropylen-Vlies | 3,63 | 1,64 |
| GORE - TEX | Polyester-Vlies 9318 | hydrophiles Polytetrafluorethylen auf Polypropylen-Vlies | 5,92 | 1,67 |
| Millipore | Durapore PVDF | hydrophilisiertes Polyvinylidenfluorid | 1,18 | 1,79 |
| Millipore | hydrophylisierte PTFE | hydrophilisiertes Polytetrafluorethylen | 2,84 | 1,72 |
| Sartorius | Membranfilter Typ 250 | hydrophiles Polyamid | 2,7 | 1,7 |

## Patentansprüche

1. Verfahren zur Isolierung von Nukleinsäuren mit den folgenden Schritten:
- Bereitstellung einer in einem Gefäß fixierten Membran aus polymerem Material
- Beschicken der Membran aus einer Richtung mit Nukleinsäuren;
- Immobilisieren der Nukleinsäuren an der Membran;
- Ablösen der immobilisierten Nukleinsäuren von der Membran mit einem Elutionsmittel; und
- Abnehmen der abgelösten Nukleinsäuren von derselben Seite der Membran von der sie der Membran zugeführt wurde,
wobei die Isolierung der Nukleinsäure an der in dem Gefäß fixierten Membran erfolgt.

2. Verfahren nach Anspruch 1, **dadurch gekennzeichnet, daß** das Beschicken von oben erfolgt.

3. Verfahren nach Anspruch 1 oder 2, **dadurch gekennzeichnet, daß** zwischen dem Immobilisierungs- und dem Ablöseschritt ein Waschen der immobilisierten Nukleinsäuren mit zumindest einem Waschpuffer erfolgt.

4. Verfahren nach Anspruch 3, **dadurch gekennzeichnet, daß** das Waschen für jeden Waschpuffer folgende Schritte umfasst:
- Aufbringen einer vorbestimmten Menge an Waschpuffer auf die Membran; und
- Durchsaugen des Waschpuffers durch die Membran.

5. Verfahren nach einem der vorstehenden Ansprüche, **dadurch gekennzeichnet, daß** das Beschicken und Immobilisieren der Nukleinsäuren folgende Schritte umfasst:
- Mischen der Nukleinsäuren mit einem Immobilisierungspuffer,
- Beschicken der Nukleinsäuren mit dem Immobiliserungspuffer auf die Membran
- Durchsaugen der füssigen Bestandteile durch die Membran.

6. Verfahren nach einem der vorstehenden Ansprüche, **dadurch gekennzeichnet, daß** zumindest einer der Schritte durch einen Automaten vollautomatisch durchgeführt wird.

7. Verfahren nach Anspruch 6, **dadurch gekennzeichnet, daß** alle Schritte des Verfahrens durch einen Automaten in gesteuerter Abfolge durchgeführt werden.

8. Verfahren nach einem der vorstehenden Ansprüche, **dadurch gekennzeichnet, daß** eine Mehrzahl von Nukleinsäureisolierungen gleichzeitig durchgeführt werden.

9. Verfahren nach einem der vorstehenden Ansprüche, **dadurch gekennzeichnet, daß** zwischen dem Ablöse- und dem Abnehmschritt zumindest einmal folgende Schritte durchgeführt werden:
- Durchführen zumindest einer chemischen Reaktion an den Nukleinsäuren;
- Immobilisieren der Nukleinsäuren an der Membran; und
- Ablösen der immobilisierten Nukleinsäuren von der Membran mit einem Elutionsmittel.

10. Verfahren nach einem der vorstehenden Ansprüche, **dadurch gekennzeichnet, daß** zum Immobilisieren der Nukleinsäuren wässerige Lösungen von Salzen der Alkali- oder Erdalkalimetalle mit Mineralsäuren eingesetzt werden.

11. Verfahren nach Anspruch 10, **dadurch gekennzeichnet, daß** zum Immobilisieren der Nukleinsäuren Alkali- oder Erdalkalihalogenide -oder -sulfate eingesetzt werden.

12. Verfahren nach Anspruch 11, **dadurch gekennzeichnet, daß** zum Immobilisieren der Nukleinsäuren Halogenide des Natriums oder Kaliums oder Magnesiumsulfat eigesetzt werden.

13. Verfahren nach einem der Ansprüche 1 bis 9, **dadurch gekennzeichnet, daß** zum Immobilisieren der Nukleinsäuren wässerige Lösungen von Salzen von ein- oder mehrbasischen oder polyfunktionellen organischen Säuren mit Alkali- oder Erdalkalimetallen eingesetzt werden.

14. Verfahren nach Anspruch 13, **dadurch gekennzeichnet, daß** zum Immobilisieren der Nukleinsäuren wässerige Lösungen von Salzen des Natriums, des Kaliums oder des Magnesiums mit organischen Dicarbonsäuren eingesetzt werden.

15. Verfahren nach Anspruch 14, **dadurch gekennzeichnet,daß** die organische Dicarbonsäure Oxalsäure, Malonsäure und/oder Bernsteinsäure ist.

16. Verfahren nach Anspruch 13, **dadurch gekennzeichnet, daß** zum Immobilisieren der Nukleinsäuren wässerige Lösungen von Salzen des Natriums oder des Kaliums mit einer Hydroxy- oder Polyhydroxycarbonsäure eingesetzt werden.

17. Verfahren nach Anpspruch 16, **dadurch gekennzeichnet, daß** die Polyhydroxycarbonsäure Zitronensäure ist.

18. Verfahren nach einem der Ansprüche 1 bis 9, **dadurch gekenneichnet, daß** zum Immobilisieren der Nukleinsäuren Hydroxylderivate von aliphatischen oder acyclischen gesättigten oder ungesättigten Kohlenwasserstoffen eingesetzt werden.

19. Verfahren nach Anspruch 18, **dadurch gekennzeichnet, daß** als Hydroxylderivate C1-C5-Alkanole eingesetzt werden.

20. Verfahren nach Anspruch 19, **dadurch gekennzeichnet, daß** als C1-C5-Alkanole Methanol, Ethanol, n-Propanol, tert.-Butanol und/oder Pentanole eingesetzt werden.

21. Verfahren nach Anspruch 18, **dadurch gekennzeichnet, daß** als Hydroxylderivat ein Aldit eingesetzt wird.

22. Verfahren nach einem der Ansprüche 1 bis 9, **dadurch gekennzeichnet, daß** zum Immobilisieren der Nukleinsäuren ein Phenol oder Polyphenol eingesetzt wird.

23. Verfahren nach Ansprüche 3 bis 22, **dadurch gekennzeichnet, daß** zum Waschen der immobilisierten Nukleinsäuren eine Salzlösung oder eine Pufferlösung gemäß einem der Ansprüche 4 bis 22 eingesetzt wird.

24. Verfahren nach einem der vorstehenden Ansprüche, **dadurch gekennzeichnet, daß** zum Ablösen der Nukleinsäuren eine wässerige Salz- oder Pufferlösung als Elutionsmittel eingesetzt wird.

25. Verfahren nach einem der Ansprüche 1 bis 23, **dadurch gekennzeichnet, daß** zum Ablösen der Nukleinsäuren Wasser als Elutionsmittel eingesetzt wird.

26. Verfahren nach einem der vorstehenden Ansprüche, **dadurch gekennzeichnet, daß** zum Immobilisieren der Nukleinsäuren chaotrope Agenzien eingesetzt werden.

27. Verfahren nach Anspruch 26, **dadurch gekennzeichnet, daß** das chaotrope Agenz ein Salz aus der Gruppe der Trichloracetate, Thiocyanate, Perchlorate, Jodide oder Guanidin-Hydrochlorid, Guanidinium-iso-thiocyanat oder Harnstoff ist.

28. Verfahren nach Anspruch 26 oder 27, **dadurch gekennzeichnet, daß** 0,01 molare bis 10 molare wässerige Lösungen der chaotropen Agenzien allein oder in Kombination mit anderen Salzen zum Immobilisieren der Nukleinsäuren eingesetzt werden.

29. Verfahren nach Anspruch 28, **dadurch gekennzeichnet, daß** 0,1 molare bis 7 molare wässerige Lösungen der chaotropen Agenzien allein oder in Kombination mit anderen Salzen zum Immobilisieren der Nukleinsäuren eingesetzt werden.

30. Verfahren nach Anspruch 29, **dadurch gekennzeichnet, daß** 0,2 molare bis 5 molare wässerige Lösungen der chaotropen Agenzien allein oder in Kombination mit anderen Salzen zum Immobilisieren der Nukleinsäuren eingesetzt werden.

31. Verfahren nach einem der Ansprüche 26 bis 30, **dadurch gekennzeichnet, daß** eine wässerige Lösung von Natriumperchlorat, Guanidinium-Hydrochlorid, Guanidinium-iso-thiocyanat, Natriumiodid und/oder Kaliumiodid zum Immobilisieren der Nukleinsäuren eingesetzt wird.

32. Verfahren nach einem der Ansprüche 1 bis 31, **dadurch gekennzeichnet, daß** die Membran eine hydrophobe Membran ist.

33. Verfahren nach Anspruch 32, **dadurch gekennzeichnet, daß** die hydrophobe Membran aus einem Polymer mit polaren Gruppen aufgebaut ist.

34. Verfahren nach Anspruch 32 oder 33, **dadurch gekennzeichnet, daß** die Membran eine hydrophile Membran mit einer hydrophobisierten Oberfläche ist.

35. Verfahren nach einem der Ansprüche 32 bis 34, **dadurch gekennzeichnet, daß** die Membran aus Nylon, Polysulfonen, Polyethersulfonen, Polycarbonaten, Polyacrylaten sowie Acrylsäurecopolymeren, Polyurethanen, Polyamiden, Polyvinylchlorid, Polyfluorocarbonaten, Polytetrafluoroethylen, Polyvinylidenfluorid, Polyvinylidendifluorid, Polyethylentetrafluoroethylen-Copolymerisaten, Polyethylenchlorotrifluoroethylen-Coplymerisaten oder Polyphenylensulfiden besteht.

36. Verfahren nach Anspruch 34 oder 35, **dadurch gekennzeichnet, daß** die Membran aus einem hydrophobisierten Nylon besteht.

37. Verfahren nach einem der Ansprüche 34 bis 36, **dadurch gekennzeichnet, daß** die Membran mit einem Hydrophobisierungsmittel aus der Gruppe der Paraffine, Wachse, Metallseifen ggf. mit Zusätzen an Aluminium bzw. Zirkoniumsalzen, quartären organische Verbindungen, Harnstoffderivate, fettstoffmodifizierten Melaminharze, Silicone, zinkorganischen Verbindungen und/oder mit Glutardialdehyd beschichtet ist.

38. Verfahren nach einem der Ansprüche 1 bis 31, **dadurch gekennzeichnet, daß** die. Membran eine hydrophile oder hydrophilisierte Membran ist.

39. Verfahren nach Anspruch 38, **dadurch gekennzeichnet, daß** die Membran aus Nylon, Polyethersulfonen, Polycarbonaten, , Polyamiden,Polyvinylchlorid, Polytetrafluoroethylenen, Polyvinylidenfluoriden,Polyester, Polyvinylidendifluoriden, Polyethylentetrafluoroethylen-Copolymerisat, einem Polyethylenchlorotrifluoroethylen-Copolymerisat oder Polyphenylensulfid besteht.

40. Verfahren nach einem der Ansprüche 1 bis 39, **dadurch gekennzeichnet, daß** die Membran einen Porendurchmesser von 0,001 bis 50 Mikrometer, vorzugsweise 0,01 bis 20 Mikrometer, besonders bevorzugt 0,05 bis. 10 Mikrometer besitzt.

41. Verfahren nach einem der vorstehenden Ansprüche, **dadurch gekennzeichnet, daß** die immobilisierung bei einem pH von 3 bis 11 erfolgt.

42. Verwendung zumindest einer in einem Gefäß fixierten Membran aus polymerem Material zum Immobilisieren von Nukleinsäuren auf einer Seite der Membran und Ablösen der Nukleinsäuren an derselben Seite zu deren Isolierung.

43. Verwendung nach Anspruch 42, **dadurch gekennzeichnet, daß** die Membran aus Nylon, Polysulfonen, Polyethersulfonen, Polycarbonaten, Polyacrylaten sowie Acrylsäurecopolymeren, Polyurethanen, Polyamiden, Polyvinylchlorid, Polyfluorocarbonaten, Polytetrafluoroethylen, Polyvinylidenfluorid, Polyvinylidendifluorid, Polyethylentetrafluoroethylen-Copolymerisaten, Polyethylenchlorodifluoroethylen-Copolymerisaten oder Polyphenylensulfid besteht.

44. Verwendung nach Anspruch 42, **dadurch gekennzeichnet, daß** die Membran eine hydrophobisierte Nylon-Membran ist.

45. Verwendung nach einem der Ansprüche 42 bis 44, **dadurch gekennzeichnet, daß** die Membran eine hydrophile Membran ist, die mit einem Hydrophobisierungsmittel aus der Gruppe der Paraffine, Wachse, Metallseifen ggf. mit Zusätzen an Aluminium bzw. Zirkoniumsalzen, quartären organische Verbindungen, Harnstoffderivate, fettstoffmodifizierten Melaminharze, Silicone, zinkorganischen Verbindungen und/oder mit Glutardialdehyd überzogen ist.

46. Verwendung nach einem der Ansprüche 42 bis 45, **dadurch gekennzeichnet, daß** eine Mehrzahl von Membranen in Isoliergefässen auf einer Multiwellplatte angeordnet sind.

## Claims

1. Process for the isolation of nucleic acids with the following steps:
- providing a membrane of a polymeric material fixed in a device
- charging of the membrane from a given direction with nucleic acids
- immobilizing the nucleic acids on the membrane
- releasing the immobilized nucleic acids from the membrane by means of an eluting agent; and
- removing the released nucleic acids from the same side of the membrane, from which they were applied to the membrane,
wherein the isolation of the nucleic acid occurs on said membrane fixed in said device.

2. Process according to claim 1, **characterized in that** the charging takes place from the top.

3. Process according to claim 1 or 2, **characterized in that**, between the immobilization and release steps, a washing of the immobilized nucleic acids with at least one washing buffer takes place.

4. Process according to claim 3, **characterized in that** the washing includes the following steps for each washing buffer:
- transfer of a predetermined amount of washing buffer to the membrane: and
- drawing the washing buffer through the membrane by suction.

5. Process according to one of the preceding claims, **characterized in that** the charging and immobilization of the nucleic acids includes the following steps:
- mixing of the nucleic acids with an immobilization buffer;
- charging of the nucleic acids with the immobilization buffer on to the membrane;
- drawing the fluid components through the membrane.

6. Process according to one of the preceding claims, **characterized in that** at least one of the steps can be carried out completely automatically by means of an automated apparatus.

7. Process according to claim 6, **characterized in that** all the steps in the process can be carried out by an automated apparatus in a guided sequence.

8. Process according to one of the preceding claims, **characterized in that** a majority of nucleic acid isolations can be carried out simultaneously.

9. Process according to one of the preceding claims, **characterized in that** between the release and the removal steps, the following steps can be carried out at least once:
- carrying out of at least one chemical reaction on the nucleic acids;
- immobilization of the nucleic acids on the membrane; and
- release of the immobilized nucleic acids from the membrane by means of an eluting agent.

10. Process according to one of the preceding claims, **characterized in that**, for the immobilization of nucleic acids, aqueous solutions of salts of alkaline and alkaline earth metals with mineral acids are used.

11. Process according to claim 10, **characterized in that**, for the immobilization of nucleic acids, alkaline or alkaline earth halogenides or sulfates are used.

12. Process according to claim 11, **characterized in that**, for the immobilization of nucleic acids, halogenides of sodium or potassium or magnesium sulfate are used.

13. Process according to one of claims 1 through 9, **characterized in that**, for the immobilization of nucleic acids, aqueous solutions of salts of monobasic or polybasic or polyfunctional organic acids with alkaline or alkaline earth metals are used.

14. Process according to claim 13, **characterized in that**, for the immobilization of nucleic acids, aqueous solutions of salts of sodium, potassium, or magnesium with organic dicarboxylic acids are used.

15. Process according to claim 14, **characterized in that** the organic dicarboxylic acid is oxalic acid, malonic acid, and/or succinic acid.

16. Process according to claim 13, **characterized in that**, for the immobilization of nucleic acids, aqueous solutions of salts of sodium or potassium with hydroxycarboxylic or polyhydroxycarboxylic acid can be used.

17. Process according to claim 16, **characterized in that** polyhydroxycarboxylic acid is citric acid.

18. Process according to claims 1 through 9, **characterized in that**, for the immobilization of nucleic acids, hydroxyl derivatives of aliphatic or acyclic saturated or unsaturated hydrocarbons are used.

19. Process according to claim 18, **characterized in that** C1-C5 alkanols are used as hydroxyl derivatives.

20. Process according to claim 19, **characterized in that** methanol, ethanol, n-propanol, tert-butanol and/or pentanols are used as C1-C5 alkanols.

21. Process according to claim 18, **characterized in that** an aldite is used as an hydroxyl derivative.

22. Process according to claims 1 through 9, **characterized in that**, for the immobilization of nucleic acids, a phenol or polyphenol is used.

23. Process according to claims 3 through 22, **characterized in that**, for the washing of immobilized nucleic acids, a salt solution or a buffer solution pursuant to one of claims 4 through 22 is used.

24. Process according to one of the preceding claims, **characterized in that** an aqueous salt or buffer solution is used as an eluting agent for the release of the nucleic acids.

25. Process according to one of claims 1 through 23, **characterized in that** for the release of nucleic acids water is used as an eluting agent.

26. Process according to one of the preceding claims, **characterized in that** chaotropic agents are used for the immobilization of the nucleic acids.

27. Process according to claim 26, **characterized in that** the chaotropic agent is a salt selected from the group consisting of trichloro-acetates, thiocyanates, perchlorates, iodides, or guanidinium hydrochloride, guanidinium isothiocyanate, or urea.

28. Process according to claims 26 or 27, **characterized in that** 0,01 M to 10 M aqueous solutions of the chaotropic agents, alone or in combination with other salts, are used for the immobilization of nucleic acids.

29. Process according to claim 28, **characterized in that** 0,1 M to 7 M aqueous solutions of the chaotropic agents, alone or in combination with other salts, are used for the immobilization of the nucleic acids.

30. Process according to claim 29, **characterized in that** 0,2 M to 5 M aqueous solutions of chaotropic agents, alone or in combination with other salts, are used for the immobilization of nucleic acids.

31. Process according to one of claims 26 through 30, **characterized in that** an aqueous solution of sodium perchlorate, guanidinium hydrochloride, guanidinium isothiocyanate, sodium iodide and/or potassium iodide are used for the immobilization of the nucleic acids.

32. Process according to one of the claims 1 through 31, **characterized in that** the membrane is a hydrophobic membrane.

33. Process according to claim 32, **characterized in that** the hydrophobic membrane is made of a polymer with polar groups.

34. Process according to claims 32 or 33, **characterized in that** the membrane is a hydrophilic membrane with a hydrophobisized surface.

35. Process according to claims 32 through 34, **characterized in that** the membrane consists of nylon, polysulfones, polyether sulfones, polycarbonates, polyacrylates, as well as acrylic acid copolymers, polyurethanes, polyamides, polyvinyl chlorides, polyfluorocarbonates, polytetrafluoroethylenes, polyvinylidene fluoride, polyvinylidene difluoride, polyethylene tetrafluoroethylene copolymerisates, polyethylene chlorotrifluoroethylene copolymerisates, or polyphenylene sulfides.

36. Process according to claims 34 or 35, **characterized in that** the membrane consists of a hydrophobisized nylon.

37. Process according to claim 34 through 36, **characterized in that** the membrane is coated with a hydrophobic coating agent selected from the group consisting of paraffins, waxes, metallic soaps, in some cases with the admixture of aluminum or zirconium salts, quaternary organic compounds, urea derivatives, lipid-modified melamine resins, silicones, organic zinc compounds, and/or glutaric dialdehyde.

38. Process according to one of the claims 1 through 31, **characterized in that** the membrane is a hydrophilic or hydrophilized membrane.

39. Process according to claim 38, **characterized in that** the membrane consists of nylon, polyether sulfones, polycarbonates, polyamides, polyvinyl chloride, polytetrafluoroethylenes, polyvinylidene fluorides, polyesters, polyvinylidene difluorides, polyethylene tetrafluoroethylene copolymerisate, a polyethylene chlorotrifluoroethylene copolymerisate, or polyphenylene sulfide.

40. Process according to one of the claims 1 through 39, **characterized in that** the pores of the membrane have a diameter of 0,001 to 50 micrometers, preferably from 0,01 to 20 micrometers, most preferably from 0,05 to 10 micrometers.

41. Process according to one of the preceding claims, **characterized in that** immobilization takes place at a pH of from 3 to 11.

42. Use of at least one membrane of polymeric material fixed in a device for the immobilization of nucleic acids on one side of the membrane and release of the nucleic acids on the same side as for their isolation.

43. Process according to claim 42, **characterized in that** the membrane consists of nylon, polysulfones, polyether sulfones, polycarbonates, polyacrylates, as well as of acrylic acid copolymers, polyurethanes, polyamides, polyvinyl chloride, polyfluorocarbonates, polytetrafluoroethylene, polyvinylidene fluoride, polyvinylidene difluoride, polyethylene tetrafluoroethylene copolymerisates, polyethylene chlorodifluoroethylene copolymerisates or polyphenylene sulfide.

44. Use according to claim 42, **characterized in that** the membrane is a hydrophobisized nylon membrane.

45. Use according to one of the claims 42 through 44, **characterized in that** the membrane is a hydrophilic membrane, which is coated with a hydrophobical coating agent selected from the group consisting of paraffins, waxes, metallic soaps, in some cases with the admixture of aluminum or zirconium salts, quaternary organic compounds, urea derivatives, lipid-modified melamine resins, silicones, organic zinc compounds, and/or glutaric dialdehyde.

46. Use according to one of claims 42 through 45, **characterized in that** a majority of membranes in isolation devices are accommodated on a multi-well plate.

## Revendications

1. Procédé d'isolement d'acides nucléiques comportant les étapes suivantes:
- préparation d'une membrane en matériau polymère fixée dans un récipient
- chargement de la membrane à partir d'une direction avec des acides nucléiques;
- immobilisation des acides nucléiques sur la membrane;
- détachement des acides nucléiques immobilisés de la membrane avec un éluant; et
- récupération des acides nucléiques détachés du même côté de la membrane que celui à partir duquel ils ont été amenés sur la membrane,
l'isolement de l'acide nucléique s'effectuant sur la membrane fixée dans le récipient.

2. Procédé selon la revendication 1, **caractérisé en ce que** le chargement se fait à partir du haut.

3. Procédé selon la revendication 1 ou 2, **caractérisé en ce que**, entre l'étape d'immobilisation et l'étape de détachement, on effectue un lavage des acides nucléiques immobilisés avec au moins un tampon de lavage.

4. Procédé selon la revendication 3, **caractérisé en ce que** le lavage comprend les étapes suivantes pour chaque tampon de lavage:
- application d'une quantité prédéterminée de tampon de lavage sur la membrane;
- aspiration du tampon de lavage à travers la membrane.

5. Procédé selon l'une des revendications précédentes, **caractérisé en ce que** le chargement et l'immobilisation des acides nucléiques comprennent les étapes suivantes:
- mélange des acides nucléiques avec un tampon d'immobilisation,
- chargement des acides nucléiques avec le tampon d'immobilisation sur la membrane,
- aspiration des constituants liquides à travers la membrane.

6. Procédé selon l'une des revendications précédentes, **caractérisé en ce qu'**au moins l'une des étapes est effectuée de manière entièrement automatique par un appareil automatique.

7. Procédé selon la revendication 6, **caractérisé en ce que** toutes les étapes du procédé sont effectuées par un appareil automatique dans un ordre contrôlé.

8. Procédé selon l'une des revendications précédentes, **caractérisé en ce qu'**une pluralité d'isolements d'acides nucléiques sont effectués simultanément.

9. Procédé selon l'une des revendications précédentes, **caractérisé en ce que**, entre l'étape de détachement et l'étape de récupération, on effectue au moins une fois les étapes suivantes:
- mise en oeuvre d'au moins une réaction chimique sur les acides nucléiques;
- immobilisation des acides nucléiques sur la membrane; et
- détachement des acides nucléiques immobilisés de la membrane avec un éluant.

10. Procédé selon l'une des revendications précédentes, **caractérisé en ce que**, pour l'immobilisation des acides nucléiques, on utilise des solutions aqueuses de sels de métaux alcalins ou de métaux alcalino-terreux avec des acides inorganiques.

11. Procédé selon la revendication 10, **caractérisé en ce que**, pour l'immobilisation des acides nucléiques, on utilise des halogénures ou des sulfates de métaux alcalins ou de métaux alcalino-terreux.

12. Procédé selon la revendication 11, **caractérisé en ce que**, pour l'immobilisation des acides nucléiques, on utilise des halogénures de sodium ou de potassium ou du sulfate de magnésium.

13. Procédé selon l'une des revendications 1 à 9, **caractérisé en ce que**, pour l'immobilisation des acides nucléiques, on utilise des solutions aqueuses de sels d'acides organiques mono- ou polybasiques ou polyfonctionnels avec des métaux alcalins ou des métaux alcalino-terreux.

14. Procédé selon la revendication 13, **caractérisé en ce que**, pour l'immobilisation des acides nucléiques, on utilise des solutions aqueuses de sels de sodium, de potassium ou de magnésium avec des acides dicarboxyliques organiques.

15. Procédé selon la revendication 14, **caractérisé en ce que** l'acide dicarboxylique organique est l'acide oxalique, l'acide malonique et/ou l'acide succinique.

16. Procédé selon la revendication 13, **caractérisé en ce que**, pour l'immobilisation des acides nucléiques, on utilise des solutions aqueuses de sels de sodium ou de potassium avec un acide hydroxy- ou polyhydroxycarboxylique.

17. Procédé selon la revendication 16, **caractérisé en ce que** l'acide polyhydroxycarboxylique est l'acide citrique.

18. Procédé selon l'une des revendications 1 à 9, **caractérisé en ce que**, pour l'immobilisation des acides nucléiques, on utilise des dérivés hydroxylés d'hydrocarbures aliphatiques ou acycliques, saturés ou insaturés.

19. Procédé selon la revendication 18, **caractérisé en ce que** l'on utilise des alcanols en C₁-C₅ comme dérivés hydroxylés.

20. Procédé selon la revendication 19, **caractérisé en ce que** l'on utilise du méthanol, de l'éthanol, du n-propanol, du tert-butanol et/ou des pentanols comme alcanols en C₁-C₅.

21. Procédé selon la revendication 18, **caractérisé en ce que** l'on utilise un alditol comme dérivé hydroxylé.

22. Procédé selon l'une des revendications 1 à 9, **caractérisé en ce que**, pour l'immobilisation des acides nucléiques, on utilise un phénol ou un polyphénol.

23. Procédé selon l'une des revendications 3 à 22, **caractérisé en ce que**, pour le lavage des acides nucléiques immobilisés, on utilise une solution de sel ou une solution de tampon selon l'une des revendications 4 à 22.

24. Procédé selon l'une des revendications précédentes, **caractérisé en ce que**, pour détacher les acides nucléiques, on utilise une solution aqueuse de sel ou de tampon comme éluant.

25. Procédé selon l'une des revendications 1 à 23, **caractérisé en ce que**, pour détacher les acides nucléiques, on utilise de l'eau comme éluant.

26. Procédé selon l'une des revendications précédentes, **caractérisé en ce que**, pour l'immobilisation des acides nucléiques, on utilise des agents chaotropes.

27. Procédé selon la revendication 26, **caractérisé en ce que** l'agent chaotrope est un sel du groupe des trichloroacétates, des thiocyanates, des perchlorates, des iodures, ou le chlorhydrate de guanidine, l'isothiocyanate de guanidinium ou l'urée.

28. Procédé selon la revendication 26 ou 27, **caractérisé en ce que** l'on utilise des solutions aqueuses 0,01 M à 10 M des agents chaotropes seuls ou en combinaison avec d'autres sels pour l'immobilisation des acides nucléiques.

29. Procédé selon la revendication 28, **caractérisé en ce que** l'on utilise des solutions aqueuses 0,1 M à 7 M des agents chaotropes seuls ou en combinaison avec d'autres sels pour l'immobilisation des acides nucléiques.

30. Procédé selon la revendication 29, **caractérisé en ce que** l'on utilise des solutions aqueuses 0,2 M à 5 M des agents chaotropes seuls ou en combinaison avec d'autres sels pour l'immobilisation des acides nucléiques.

31. Procédé selon l'une des revendications 26 à 30, **caractérisé en ce que** l'on utilise une solution aqueuse de perchlorate de sodium, de chlorhydrate de guanidinium, d'isothiocyanate de guanidinium, d'iodure de sodium et/ou d'iodure de potassium pour l'immobilisation des acides nucléiques.

32. Procédé selon l'une des revendications 1 à 31, **caractérisé en ce que** la membrane est une membrane hydrophobe.

33. Procédé selon la revendication 32, **caractérisé en ce que** la membrane hydrophobe est constituée d'un polymère ayant des groupes polaires.

34. Procédé selon la revendication 32 ou 33, **caractérisé en ce que** la membrane est une membrane hydrophile ayant une surface rendue hydrophobe.

35. Procédé selon l'une des revendications 32 à 34, **caractérisé en ce que** la membrane est constituée de nylon, de polysulfone, de polyéthersulfone, de polycarbonate, de polyacrylate ainsi que des copolymères d'acide acrylique, de polyuréthane, de polyamide, de poly(chlorure de vinyle), de polyfluorocarbonate, de polytétrafluoroéthylène, de poly(fluorure de vinylidène), de poly(difluorure de vinylidène), de copolymère poly(éthylène-tétrafluoroéthylène), de copolymère poly(éthylène-chlorotrifluoroéthylène) ou de poly(sulfure de phénylène).

36. Procédé selon la revendication 34 ou 35, **caractérisé en ce que** la membrane est constituée de nylon rendu hydrophobe.

37. Procédé selon l'une des revendications 34 à 36, **caractérisé en ce que** la membrane est revêtue d'un agent d'hydrophobisation du groupe des paraffines, des cires, des savons métalliques éventuellement avec des additions de sels d'aluminium ou de zirconium, des composés organiques quaternaires, des dérivés d'urée, des résines mélamine modifiées avec des matières grasses, des silicones, des composés organiques du zinc et/ou de glutaraldéhyde.

38. Procédé selon l'une des revendications 1 à 31, **caractérisé en ce que** la membrane est une membrane hydrophile ou rendue hydrophile.

39. Procédé selon la revendication 38, **caractérisé en ce que** la membrane est constituée de nylon, de polyéthersulfone, de polycarbonate, de polyamide, de poly(chlorure de vinyle), de polytétrafluoroéthylène, de poly(fluorure de vinylidène), de polyester, de poly(difluorure de vinylidène), de copolymère poly(éthylène-tétrafluoroéthylène), d'un copolymère poly(éthylène-chlorotrifluoroéthylène) ou de poly(sulfure de phénylène).

40. Procédé selon l'une des revendications 1 à 39, **caractérisé en ce que** la membrane a un diamètre de pores de 0,001 à 50 µm, de préférence de 0,01 à 20 µm, de façon particulièrement préférée de 0,05 à 10 µm.

41. Procédé selon l'une des revendications précédentes, **caractérisé en ce que** l'immobilisation s'effectue à un pH de 3 à 11.

42. Utilisation d'au moins une membrane de matériau polymère fixée dans un récipient pour l'immobilisation d'acides nucléiques sur un côté de la membrane et le détachement des acides nucléiques du même côté pour son isolement.

43. Utilisation selon la revendication 42, **caractérisée en ce que** la membrane est constituée de nylon, de polysulfone, de polyéthersulfone, de polycarbonate, de polyacrylate ainsi que des copolymères d'acide acrylique, de polyuréthane, de polyamide, de poly(chlorure de vinyle), de polyfluorocarbonate, de polytétrafluoroéthylène, de poly(fluorure de vinylidène), de poly(difluorure de vinylidène), de copolymère poly(éthylène-tétrafluoroéthylène), de copolymère poly(éthylène-chlorodifluoroéthylène) ou de poly(sulfure de phénylène).

44. Utilisation selon la revendication 42, **caractérisée en ce que** la membrane est une membrane de nylon rendue hydrophobe.

45. Utilisation selon l'une des revendications 42 à 44, **caractérisée en ce que** la membrane est une membrane hydrophile qui est revêtue d'un agent d'hydrophobisation du groupe des paraffines, des cires, des savons métalliques éventuellement avec des additions de sels d'aluminium ou de zirconium, des composés organiques quaternaires, des dérivés d'urée, des résines mélamine modifiées avec des matières grasses, des silicones, des composés organiques du zinc et/ou de glutaraldéhyde.

46. Utilisation selon l'une des revendications 42 à 45, **caractérisée en ce qu'**une pluralité de membranes sont disposées dans des réservoirs d'isolement sur une plaque multipuits.
